# EUROPEAN PATENT SPECIFICATION

(11) **EP 3 132 025 B1**
(45) Date of publication and mention of the grant of the patent: **30.08.2023**
(21) Application number: 15780038.4
(22) Date of filing: 13.04.2015
(51) Int. Cl.: C12N 5/10

(54) **METHODS AND COMPOSITIONS FOR MODIFYING GENOMIC DNA**
VERFAHREN UND ZUSAMMENSETZUNGEN ZUR MODIFIZIERUNG VON GENOMISCHER DNA
PROCÉDÉS ET COMPOSITIONS PERMETTANT DE MODIFIER L'ADN GÉNOMIQUE

(30) Priority: 14.04.2014 US 201461979178 P; 12.11.2014 US 201462078706 P
(43) Date of publication of application: 22.02.2017
(73) Proprietor: Maxcyte, Inc., Rockville, MD 20850 (US)
(72) Inventor: LI, Linhong, Gaithersburg, MD 20878 (US); PESHWA, Madhusudan, Gaithersburg, MD 20878 (US)
(74) Representative: Nottrott, Stephanie
(86) International application number: PCT/US2015/025523
(87) International publication number: WO 2015/160683

(56) References cited:
- WO-A1-2012/012738
- WO-A1-2013/139994
- WO-A1-2014/144237
- WO-A2-2007/118208
- US-A1- 2003 032 175
- US-A1- 2008 138 877
- US-A1- 2014 065 616
- K. MUSUNURU: "Genome editing of human pluripotent stem cells to generate human cellular disease models", DISEASE MODELS & MECHANISMS, vol. 6, no. 4, 10 June 2013 (2013-06-10), pages 896-904, XP055373050, GB ISSN: 1754-8403, DOI: 10.1242/dmm.012054
- RAN F ANN ET AL: "Genome engineering using the CRISPR-Cas9 system.", NATURE PROTOCOLS NOV 2013, vol. 8, no. 11, November 2013 (2013-11), pages 2281-2308, XP002772991, ISSN: 1750-2799
- LI LINHONG ET AL: "Genomic Editing of Human Hematopoietic Stem Cells Using Non-Viral, Clinical Scale, cGMP Platform and Messenger RNA (mRNA) Encoding Nucleases", MOLECULAR THERAPY, vol. 22, no. Suppl. 1, May 2014 (2014-05), page S278, XP002772992, & 17TH ANNUAL MEETING OF THE AMERICAN-SOCIETY-OF-GENE-AND-CELL-THERAPY (ASGCT); WASHINGTON, DC, USA; MAY 21 -24, 2014

## Description

### 1. Field of the Invention

The present invention relates generally to the field of biotechnology. More particularly, it concerns novel methods and compositions for modifying genomic DNA.

### 2. Description of Related Art

Targeted genome engineering involves editing or altering endogenous DNA in a directed manner at a specific site along the DNA within the cell. Despite the tremendous potential of gene repair and homology-directed gene alteration, current genome engineering approaches provide very low efficiency of repair or editing and have the potential to introduce harmful or undesired DNA sequences and outcomes.

The modification of the endogenous genomic sequence may provide advanced therapeutic applications as well as advanced research methods. Currently, the most common method for disruption of gene function *in vitro* is by RNA interference (RNAi). However, this approach has limitations. For example, RNAi can exhibit significant off-target effects and toxicity. Furthermore RNAi is involved in the cellular mechanisms of many endogenous processes, and artificially enacting a mechanism, such as RNAi, that may very well be involved in a pathway of interest, can lead to misleading or false results. An efficient and non-toxic mechanism of modifying the genomic sequence of a cell would be a more precise method for gene knock-down.

An efficient and non-toxic method of modifying endogenous genomic sequences may also provide advances in ex vivo therapy, since one could isolate cells from a patient, modify the genome to correct a mutation, and transplant the patient's own cells back in to achieve a therapeutic effect. Current methods are either too inefficient or too toxic to achieve these results. There is need in the field for a technology that allows for site-directed genomic DNA modification that is efficient, non-toxic, and stable

### SUMMARY OF THE INVENTION

Compositions and methods concern the sequence modification or amendment of an endogenous target genomic DNA sequence. Certain aspects relate to a method for for site-specific sequence modification of a target genomic DNA region in cells comprising: transfecting the cells by electroporation with a composition comprising (a) a DNA oligo and (b) a DNA digesting agent; wherein the DNA oligo comprises: (i) a homologous region comprising DNA sequence homologous to the target genomic DNA region; and (ii) a sequence modification region; and wherein the genomic DNA sequence is modified specifically at the target genomic DNA region.

A further aspect relates to a method for site-specific sequence modification of a target genomic DNA region in cells comprising: transfecting the stem cells by electroporation with a composition comprising (a) a DNA oligo having 100 nucleotides or less and (b) a DNA digesting agent encoded on an RNA; wherein the DNA oligo comprises: (i) a homologous region comprising DNA sequence homologous to the target genomic DNA region; and (ii) a sequence modification region; and wherein the genomic DNA sequence is modified specifically at the target genomic DNA region, and wherein the cells are stem cells or their progeny. In some embodiments, the cells are primary cells. The term "primary" as used herein refers to cells that are not immortalized and taken directly from living tissue. These cells have undergone very few population doublings and are therefore more representative of the main functional component of the tissue from which they are derived in comparison to continuous (tumor or artificially immortalized) cell lines thus representing a more representative model to the in vivo state.

The term "sequence modification" or "DNA amendment" is a change to the DNA sequence and can include an addition, a change, or a deletion to or of the endogenous genomic DNA sequence. For example, for a target genomic sequence, the donor DNA comprises a sequence complementary, identical, or homologous to the target genomic sequence and a sequence modification or amendment region. The a sequence modification region is typically located between the homologous ends. The sequence modification is not complementary or has a low degree of homology to the target genomic sequence and contains an alteration of the target genomic sequence.

"Homology" or "identity" or "similarity" refers to sequence similarity between two peptides or between two nucleic acid molecules. The term "homologous region" refers to a region on the donor DNA with a certain degree of homology with the target genomic DNA sequence. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, though preferably less than 25% identity, with one of the sequences of the present invention.

A polynucleotide or polynucleotide region (or a polypeptide or polypeptide region) has a certain percentage (for example, at least or at most 60%, 65%, 70%, 75%, 80%, 85%, 90%, 95%, 98% or 99%, or any range derivable therein) of "sequence identity" or "homology" to another sequence means that, when aligned, that percentage of bases (or amino acids) are the same in comparing the two sequences. This alignment and the percent homology or sequence identity can be determined using software programs known in the art, for example those described in Ausubel et al. eds. (2007) Current Protocols in Molecular Biology.

In some embodiments, the oligo is single-stranded. It is contemplated that a single-stranded oligo will increase tolerance of the cell to the DNA and reduce DNA-induced toxicity of the cell.

In certain embodiments, the homologous region of the donor DNA is 100% homologous or is identical to the target genomic sequence. In further embodiments, the homologous region of the donor DNA is 85, 90, 95, or 99% homologous.

In certain embodiments, the donor DNA comprises at least or at most 10, 12, 14, 16, 18, 20, 22, 24, 26, 28, 30, 32, 34, 36, 38, 40, 42, 44, 46, 48, 50, 75, 100, 150 and 200 residues of nucleic acid sequence (or any range derivable therein) that is homologous to the target genomic DNA sequence. In specific embodiments, the donor DNA comprises at least about 10 or at least about 15 or at least about 20 nucleic acids of sequence that are identical to the genomic DNA sequence. In this context, the term "identical sequence" refers to sequence that exactly matches the sequence of the genomic DNA. The identical sequence may be in a region that is on the 5' end of the DNA sequence modification and in a region that is on the 3' end of a DNA sequence modification. By way of illustrative example, when the donor DNA comprises at least 10 nucleic acids of homologous sequences, the donor DNA may comprise, for example, 5 nucleic acids of homologous sequence on each side of the sequence modification. Similarly, donor DNA comprising 10 nucleic acids of homologous sequences may comprise, for example, 5 nucleic acids of complimentary sequence on each side of the sequence modification.

The term "complementary" as used herein refers to Watson-Crick base pairing between nucleotides and specifically refers to nucleotides hydrogen bonded to one another with thymine or uracil residues linked to adenine residues by two hydrogen bonds and cytosine and guanine residues linked by three hydrogen bonds. In general, a nucleic acid includes a nucleotide sequence described as having a "percent complementarity" to a specified second nucleotide sequence. For example, a nucleotide sequence may have 80%, 90%, or 100% complementarity to a specified second nucleotide sequence, indicating that 8 of 10, 9 of 10 or 10 of 10 nucleotides of a sequence are complementary to the specified second nucleotide sequence. For instance, the nucleotide sequence 3'-TCGA-5' is 100% complementary to the nucleotide sequence 5'-AGCT-3'. Further, the nucleotide sequence 3'-TCGA- is 100% complementary to a region of the nucleotide sequence 5'-TTAGCTGG-3'. It will be recognized by one of skill in the art that two complementary nucleotide sequences include a sense strand and an antisense strand.

The term "transfecting" refers to a methods for introducing bio-active materials, such as nucleic acids, proteins, enzymes, or small molecules, into a cell. The nucleic acids may be DNA, delivered as plasmid or oligomer, and/or RNA or combinations thereof.

The term "electroporation" refers to a method of transfection in which an externally applied electrical field is applied to the cell. In certain embodiments, the electroporation method used is static electroporation.

In certain embodiments, cells are electroporated using flow electroporation. Flow electroporation involves: transferring a suspension of cells and loading molecules into an apparatus comprised of a fluid chamber or fluid flow path; the said fluid chamber or fluid flow path being comprised of electrodes disposed along sides of the fluid chamber or fluid flow path and configured to subject biological particles within the fluid chamber fluid flow path to an electric field suitable for electroporation; and transferring the electroporated cell suspension out of the apparatus. The term "flow electroporation" refers to electroporation of cells within a fluid chamber flow path. This method is particularly effective for large scale volume of cells. Static electroporation, by contrast, involves electroporation of a set and limited volume of cells due to constraints associated with moving electricity across liquid and the distance between opposing electrodes.

In certain aspects, transfecting the expression construct into cells comprises flowing a suspension of the cells through an electric field in a flow chamber, the electric field being produced by opposing oppositely charged electrodes at least partially defining the flow chamber, wherein thermal resistance of the flow chamber is less than approximately 10° C per Watt. In other certain aspects transfecting the cells comprises employing a flow electroporation device comprising a chamber for containing a suspension of cells to be electroporated; the chamber being at least partially defined by opposing oppositely chargeable electrodes; and wherein the thermal resistance of the chamber is less than approximately 10° C per Watt.

In certain aspects, transfecting the expression construct into cells comprises electroporating or exposing a suspension of the cells to an electric field in a chamber, the electric field being produced by opposing oppositely charged electrodes at least partially defining the chamber, wherein thermal resistance of the chamber is less than approximately 10° C per Watt. In other certain aspects transfecting the cells comprises employing an electroporation device comprising a chamber for containing a suspension of cells to be electroporated; the chamber being at least partially defined by opposing oppositely chargeable electrodes; and wherein the thermal resistance of the chamber is less than approximately 10° C per Watt.

In certain aspects, the thermal resistance of the chamber is approximately 0.1° C per Watt to 10° C per Watt. For example, the thermal resistance of the chamber may be approximately 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10 °C per Watt, or any thermal resistance derivable therein.

The opposing oppositely chargeable electrodes may be spaced from each other at least 1 mm, at least 2 mm, at least 3 mm, or any distance or range derivable therein. In any of the disclosed embodiments, the chamber may have a ratio of combined electrode surface in contact with buffer to the distance between the electrodes of approximately 1 to 100 cm. For example, the ratio may be approximately 1 to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 cm, or any value or range derivable therein. In certain aspects, the chamber has a ratio of combined electrode surface in contact with buffer to the distance between the electrodes of approximately 1 to 100 cm, and the opposing oppositely chargeable electrodes are spaced from each other at least 1 mm. In other aspects, the chamber has a ratio of combined electrode surface in contact with buffer to the distance between the electrodes of approximately 1 to 100 cm, and the opposing oppositely chargeable electrodes are spaced from each other at least 3 mm. In even further aspects, the chamber has a ratio of combined electrode surface in contact with buffer to the distance between the electrodes of approximately 1 to 100 cm, and the opposing oppositely chargeable electrodes are spaced from each other approximately 3 mm to approximately 2 cm. For example, the opposing oppositely chargeable electrodes may be spaced from each other approximately 3, 4, 5, 6, 7, 8, 9, or 10 mm, or any distance derivable therein, or the opposing oppositely chargeable electrodes may be spaced from each other approximately 1.0, 1.1, 1.2, 1.3, 1.4, 1.5, 1.6, 1.7, 1.8, 1.9, or 2.0 cm, or any distance derivable therein. In some aspects of these embodiments, the cells electroporated are not substantially thermally degraded thereby.

In any of the disclosed embodiments, the chamber may have a ratio of combined electrode surface in contact with buffer to the distance between the electrodes of approximately 1 to 100 cm. For example, the ratio may be approximately 1 to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 cm, or any value or range derivable therein. In certain aspects, the chamber has a ratio of combined electrode surface in contact with buffer to the distance between the electrodes of approximately 1 to 100 cm, and the opposing oppositely chargeable electrodes are spaced from each other at least 1 mm. In other aspects, the chamber has a ratio of combined electrode surface in contact with buffer to the distance between the electrodes of approximately 1 to 100 cm, and the opposing oppositely chargeable electrodes are spaced from each other at least 3 mm. In even further aspects, the chamber has a ratio of combined electrode surface in contact with buffer to the distance between the electrodes of approximately 1 to 100 cm, and the opposing oppositely chargeable electrodes are spaced from each other approximately 3 mm to approximately 2 cm. For example, the opposing oppositely chargeable electrodes may be spaced from each other approximately 3, 4, 5, 6, 7, 8, 9, or 10 mm, or any distance derivable therein, or the opposing oppositely chargeable electrodes may be spaced from each other approximately 1.0, 1.1, 1.2, 1.3, 1.4, 1.5, 1.6, 1.7, 1.8, 1.9, or 2.0 cm, or any distance derivable therein. In some aspects of these embodiments, the cells electroporated are not substantially thermally degraded thereby.

In any of the disclosed embodiments, the device may further comprise a cooling element to dissipate heat. For example, the cooling element may comprise a thermoelectric cooling element. As another example, the cooling element may comprise a cooling fluid flowing in contact with the electrode. As yet another example, the cooling element may comprise a heat sink operatively associated with the electrode. The heat resistance of the chamber may be less than approximately 3° C per Watt. In some embodiments, the heat resistance of the chamber is between approximately 0.5° C per Watt and 4° C per Watt, or the heat resistance of the chamber is between approximately 1° C per Watt and 3° C per Watt. For example, the heat resistance of the chamber may be approximately 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, or 4.0° C per Watt, or any value derivable therein.

In certain methods involving transfecting cells by electroporation, the method involves exposing a suspension of cells to an electric field having a strength of greater than 0.5 kV/cm. For example, the electric field may have a strength of greater than approximately 3.5 kV/cm. In certain aspects the electric field has a strength of greater than approximately 0.5, 1.0, 1.5, 2.0, 2.5, 30, or 3.5 kV/cm, or any value derivable therein.

In some embodiments, transfecting the cells comprises employing a flow electroporation device comprising: walls defining a flow channel having an electroporation zone configured to receive and to transiently contain a continuous flow of a suspension of cells to be electroporated; an inlet flow portal in fluid communication with the flow channel, whereby the suspension can be introduced into the flow channel through the inlet flow portal; an outlet flow portal in fluid communication with the flow channel, whereby the suspension can be withdrawn from the flow channel through the outlet portal; the walls defining the flow channel within the electroporation zone comprising a first electrode forming a substantial portion of a first wall of the flow channel and a second electrode forming a substantial portion of a second wall of the flow channel opposite the first wall, the first and second electrodes being such that when placed in electrical communication with a source of electrical energy an electric field is formed therebetween through which the suspension can flow; and wherein the thermal resistance of the flow channel is less than approximately 10° C per Watt.

In certain such embodiments, the first and second electrodes or opposing oppositely chargeable electrodes are spaced from each other at least 1 mm. Moreover, the chamber may have a ratio of combined electrode surface in contact with buffer to the distance between the electrodes of approximately 1 to 100 cm. In particular embodiments, the chamber may have a ratio of combined electrode surface in contact with buffer to the distance between the electrodes of approximately 1 to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 cm, or any value or range derivable therein. In certain embodiments, the cells electroporated by the electroporation methods described herein are not substantially thermally degraded thereby. In certain embodiments described herein, the chamber is a flow chamber.

In some aspects, the electroporation device comprises a chamber for containing a suspension of cells to be electroporated; the chamber being at least partially defined by opposing oppositely chargeable electrodes; and wherein the chamber has a ratio of combined electrode surface in contact with buffer to the distance between the electrodes of approximately 1 to 100 cm. In particular aspects, the ratio is approximately 1 to 70 cm. In other particular aspects, the ratio is approximately 1 to 50 cm. For example, the ratio may be approximately 1 to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 cm, or any value derivable therein. In certain embodiments described herein, the chamber is a flow chamber.

In some embodiments, the flow electroporation device comprises walls defining a flow channel configured to receive and to transiently contain a continuous flow of a suspension of cells to be electroporated; an inlet flow portal in fluid communication with the flow channel, whereby the suspension can be introduced into the flow channel through the inlet flow portal; an outlet flow portal in fluid communication with the flow channel, whereby the suspension can be withdrawn from the flow channel through the outlet portal; the walls defining the flow channel comprising a first electrode forming at least a portion of a first wall of the flow channel and a second electrode forming at least a portion of a second wall of the flow channel opposite the first wall, the first and second electrodes being such that when placed in electrical communication with a source of electrical energy an electric field is formed therebetween through which the suspension can flow; and wherein the thermal resistance of the flow channel is less than approximately 10° C per Watt. In certain aspects, the thermal resistance of the flow channel is approximately 0.1° C per Watt to 10° C per Watt. For example, the thermal resistance of the flow channel may be approximately 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10 °C per Watt, or any thermal resistance derivable therein. The first and second electrodes may be spaced from each other at least 1 mm, at least 2 mm, at least 3 mm, or any distance or range derivable therein. In any of the disclosed embodiments, the flow chamber may have a ratio of combined electrode surface in contact with buffer to the distance between the electrodes of approximately 1 to 100 cm. For example, the ratio may be approximately 1 to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 cm, or any value or range derivable therein. In certain aspects, the flow chamber has a ratio of combined electrode surface in contact with buffer to the distance between the electrodes of approximately 1 to 100 cm, and the first and second electrodes are spaced from each other at least 1 mm. In other aspects, the flow chamber has a ratio of combined electrode surface in contact with buffer to the distance between the electrodes of approximately 1 to 100 cm, and the first and second electrodes are spaced from each other at least 3 mm. In even further aspects, the flow chamber has a ratio of combined electrode surface in contact with buffer to the distance between the electrodes of approximately 1 to 100 cm, and the first and second electrodes are spaced from each other approximately 3 mm to approximately 2 cm. For example, the first and second electrodes may be spaced from each other approximately 3, 4, 5, 6, 7, 8, 9, or 10 mm, or any distance derivable therein, or the first and second electrodes may be spaced from each other approximately 1.0, 1.1, 1.2, 1.3, 1.4, 1.5, 1.6, 1.7, 1.8, 1.9, or 2.0 cm, or any distance derivable therein. In some aspects of these embodiments, the cells electroporated in the flow channel are not substantially thermally degraded thereby.

In certain disclosed methods and devices, the thermal resistance of the chamber is approximately 0.1° C per Watt to approximately 4° C per Watt. In some aspects, the thermal resistance of the chamber is approximately 1.5° C per Watt to approximately 2.5° C per Watt. For example, the thermal resistance of the chamber may be approximately 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, or 4.0° C per Watt, or any resistance derivable therein.

In certain disclosed methods and devices, the flow electroporation device comprises: walls defining a flow channel configured to receive and to transiently contain a continuous flow of a suspension comprising particles; an inlet flow portal in fluid communication with the flow channel, whereby the suspension can be introduced into the flow channel through the inlet flow portal; an outlet flow portal in fluid communication with the flow channel, whereby the suspension can be withdrawn from the flow channel through the outlet flow portal; the walls defining the flow channel comprising a first electrode plate forming a first wall of the flow channel and a second electrode plate forming a second wall of the flow channel opposite the first wall; wherein the area of the electrodes contact with the suspension, and the distance between the electrodes is chosen so that the thermal resistance of the flow channel is less than approximately 4° C per Watt; the paired electrodes placed in electrical communication with a source of electrical energy, whereby an electrical field is formed between the electrodes; whereby the suspension of the particles flowing through the flow channel can be subjected to an electrical field formed between the electrodes. In certain aspects, the electrode plates defining the flow channel further comprise a gasket formed from an electrically non-conductive material and disposed between the first and second electrode plates to maintain the electrode plates in spaced-apart relation, the gasket defining a channel therein forming opposed side walls of the flow channel. The gasket may, for example, form a seal with each of the first and second electrode plates. In some embodiments, the device comprises a plurality of flow channels, and the gasket comprises a plurality of channels forming opposed side walls of each of the plurality of channels. In some aspects, one of the inlet flow portal and the outlet flow portal comprises a bore formed in one of the electrode plates and in fluid communication with the flow channel. The other of the inlet flow portal and the outlet flow portal may comprise a bore formed in the one of the electrode plates and in fluid communication with the flow channel. In certain aspects, the inlet flow portal and the outlet flow portal comprise a bore formed in the other of the electrode plates and in fluid communication with the flow channel. In any of the disclosed embodiments, the device may further comprise a cooling element operatively associated with the flow channel to dissipate heat. For example, the cooling element may comprise a thermoelectric cooling element. As another example, the cooling element may comprise a cooling fluid flowing in contact with the electrode. As yet another example, the cooling element may comprise a heat sink operatively associated with the electrode. The heat resistance of the flow channel may be less than approximately 3° C per Watt. In some embodiments, the heat resistance of the flow channel is between approximately 0.5° C per Watt and 4° C per Watt, or the heat resistance of the flow channel is between approximately 1° C per Watt and 3° C per Watt. For example, the heat resistance of the flow channel may be approximately 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, or 4.0° C per Watt, or any value derivable therein.

In certain disclosed methods and devices, the first electrode may comprise an elongated, electrically conductive structure, wherein the second electrode comprises a tubular, electrically conductive structure; wherein the electrodes are concentrically arranged such that the second, tubular electrode surrounds the first electrode in spaced-apart relation thereto; and wherein the flow channel is disposed within an annular space defined between the first and second electrodes. The electrodes may form at least a portion of the walls defining the flow channel. In some embodiments, concentric annular spacers for maintaining the first and second electrodes are in spaced-apart, concentric relation. In certain aspects, the device is arranged in series or in parallel with a second, like device.

In certain methods involving transfecting cells by flow electroporation, the flow channel has a thermal resistance of less than approximately 10° C per Watt. In some methods involving transfecting the cells by flow electroporation, the method involves flowing a suspension of cells to be electroporated through a flow channel and exposing the suspension of to an electric field while flowing through the flow channel, the electric field having a strength of greater than 0.5 kV/cm. For example, the electric field may have a strength of greater than approximately 3.5 kV/cm. In certain aspects the electric field has a strength of greater than approximately 0.5, 1.0, 1.5, 2.0, 2.5, 3.0, or 3.5 kV/cm, or any value derivable therein.

In the disclosed embodiments regarding the flow electroporation device, it is specifically contemplated that parameters and parameter ranges described for flow electroporation are applicable to static electroporation devices used in the methods described herein. In specific embodiments, flow electroporation is used and static electroporation or non-flow electroporation is excluded. In a further specific embodiment, static electroporation is used and flow electroporation is excluded.

Any of the disclosed methods may include a step employing limiting dilution of the transfected cells to obtain single cell colonies. As used herein, the term "limiting dilution" refers to the process of significantly diluting a cell culture, with the goal of achieving a single cell in each culture. When such an isolated, single cell reproduces, the resulting culture will contain only clones of the original cell. For example, a multi-well plate may be used to obtain single cell cultures or colonies. For example, limiting dilution may be employed for a patient cell derived iPS study (*e.g.* for repair of sickle cell patients). iPS cells, using limited dilution approach, can be modified to a corrected hemoglobin-expressing cell, isolated, and expanded for administration to the patient.

In any of the disclosed methods, a step may be employed comprising expanding a clonal isolated and selected cell to produce clonal cells with a particular genomic DNA sequence modification.

In disclosed methods involving the expansion of a clonal isolated cell, the expansion may be for large scale manufacturing. For example, the cells may be expanded in a volume of greater than 1 L, or the cells may be expanded in a volume of greater than 3 L. In certain aspects, the cells are expanded in a volume of greater than 1.0, 1.5, 2.0, 2.5, or 3.0 L, or any value derivable therein.

In any of the disclosed methods, a further step may be employed comprising freezing transfected and selected or screened cells. An even further step may also be employed, wherein previously frozen transfected and selected/screened cells are expanded.

In the disclosed methods, the cell culture may include any additional ingredients known to those of ordinary skill in the art, as would be readily selected by those of ordinary skill in the art based on the type of cell that is cultured. For example, the cells may be cultured in sodium butyrate or comparable salt.

In the disclosed methods, a further step may be employed comprising expanding a clonal isolated and selected or screened cell to produce clonal cells having a genomic DNA sequence modification.

Further aspects relate to a method for producing a stable cell line comprising a genomic DNA sequence modification or amendment of a target genomic DNA sequence, the method comprising: transfecting the cells by electroporation with a composition comprising (a) a DNA oligo and (b) a digesting agent; wherein the donor DNA comprises: (i) a homologous region comprising nucleic acid sequence homologous to the target genomic DNA region; and (ii) a sequence modification region; and screening transfected cells for the genomic DNA sequence modification at the target genomic DNA region; isolating screened transfected cells by limiting dilution to obtain clonal cells; expanding isolated transfected cells to produce a stable cell line comprising the genomic DNA sequence modification.

The disclosure also provides for a cell line or electroporated cell produced by the methods described herein.

A further aspect relates to a method of treating a subject having or suspected of having a disease or condition by administering an effective amount of a cell line or of electroporated cells produced by the methods described herein.

It is specifically contemplated that embodiments described herein may be excluded. It is further contemplated that, when a range is described, certain ranges may be excluded.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

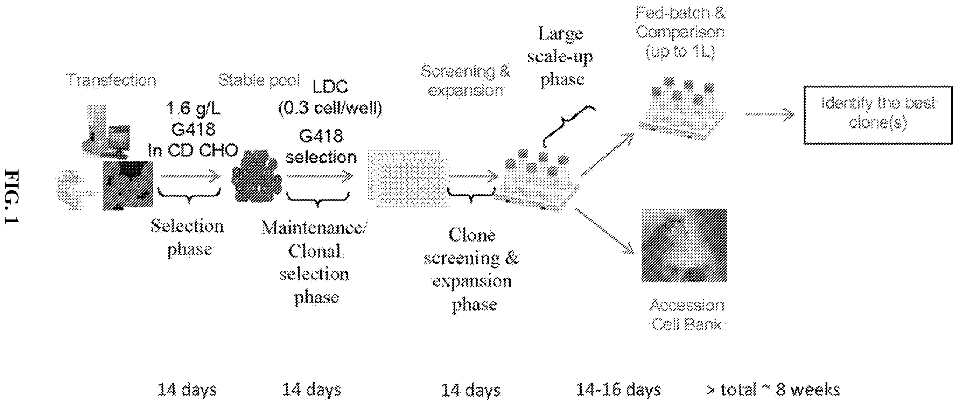

**FIG. 1****: The stable cell line development process with Maxcyte STX static and flow electroporation transfection technology.** Figure depicts work flow of stable cell generation. After electroporation, cells may be cultured for some period of time without selection to allow for recovery from the electroporation procedure (not depicted in figure). After electroporation, cells are selected for by culturing cells in the presence of a selection agent (selection phase). After the selection phase, cells are cultured at lower density in the presence of selection agent to enable limiting dilution cloning (maintenance/clonal selection phase). After the generation of clonal populations, clones are screened for exogenous polypeptide expression and expanded (clonal screening and expansion phase). After screening, clones with desired activity are grown on larger scale for production purposes (large scale-up phase) or submitted to long-term storage such as cryopreservation.

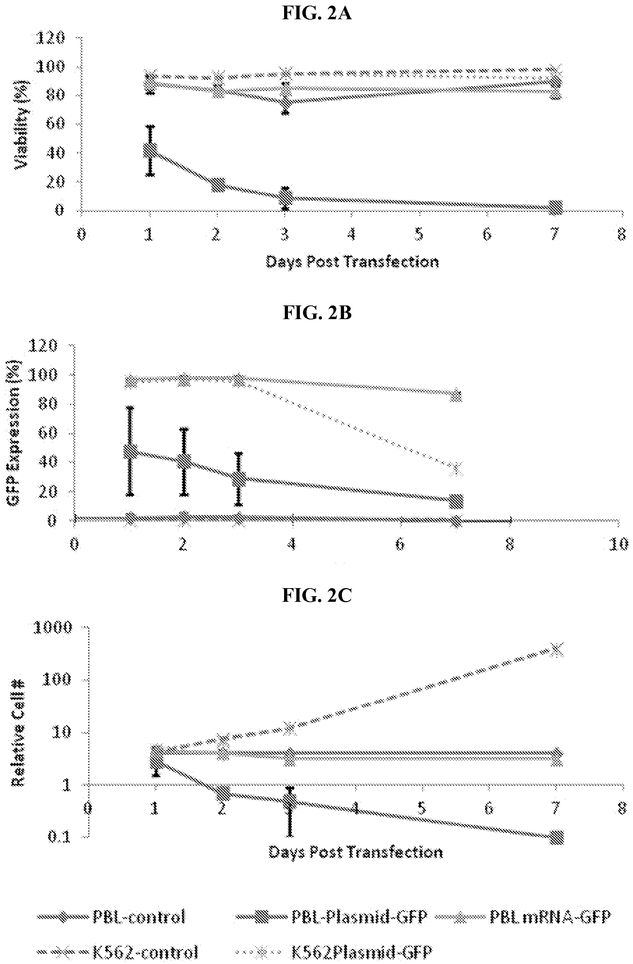

**FIG. 2A****-C: DNA transfection has differentiated cytotoxicity on cells.** Shown is in FIG. 2 is the viability of DNA and mRNA transfected peripheral blood lymphoctyes (PBL) and K562 cells (FIG. 2A), the GFP expression of DNA and mRNA transfected PBL and K562 cells (FIG. 2B), and the cell number of DNA and mRNA transfected PBL and K562 cells (FIG. 2C). The data demonstrates that DNA transfection does not cause cytotoxicity to K562, but does induce strong cytotoxicity in resting PBLs.

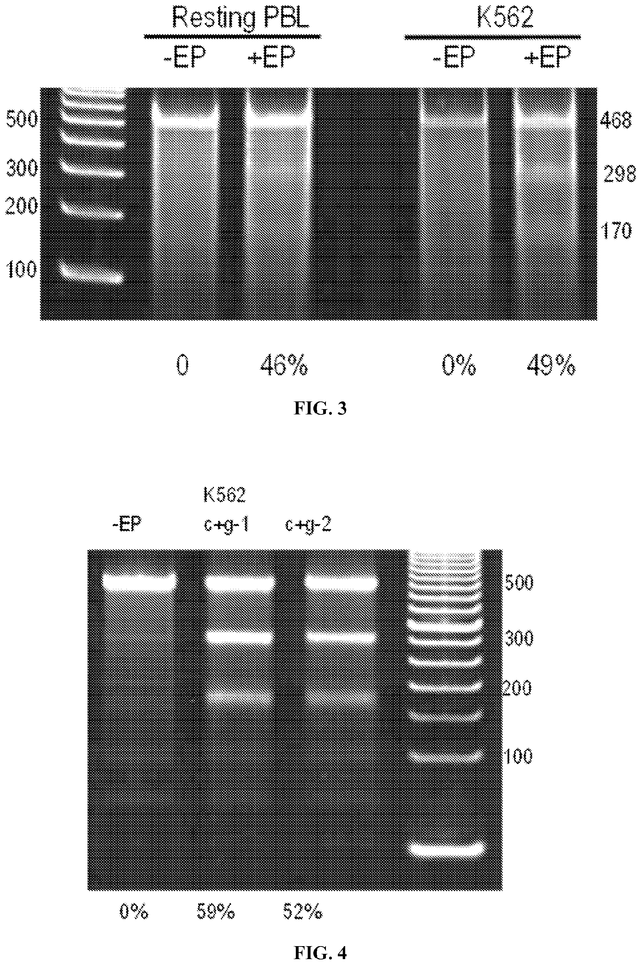

**FIG. 3****: mRNA-CRISPR transfection induced genomic DNA editing at AAVS1 site of K562 and PBL.** Depicted in FIG. 3 is a comparison of gene editing by Cel-1 assay of resting PBL cells to K562 cells. Cells were either not electroporated (-EP) or electroporated (+EP) with mRNA-CRISPRs (cas9 and gRNA respectively). The samples in this electrophoresis gel were loaded as follows: lane 1: marker; lane 2 -EP of PBL; lane 3: +EP of PBL; lane 4: -EP of K562; lane 5: +EP of K562.The cut products of a corrected AAVS-1 site are 298 and 170 basepairs, and the parental band is 468 base pairs. The editing rate was calculated as (density of digested bands)/[(density of digested bands + density of parental band). The resting electroporated resting PBL and K562 cells showed an editing rate of 46 and 49%, respectively.
**FIG. 4****: mRNA-CRISPR transfection induced genomic DNA editing at AAVS1 site of K562.** Depicted in FIG. 4 is an electrophoresis gel of a duplicated experimental result showing the consistency of the gene editing by electroporation of cells with mRNA-CRISPR (Cas9 and guide RNAs), which induced DNA editing of 59 and 52% respectively, by Cel-1 assay. The cut products of a corrected AAVS-1 site are 298 and 170 basepairs, and the parental band is 468 base pairs. The editing rate was calculated as (density of digested bands)/[(density of digested bands + density of parental band).

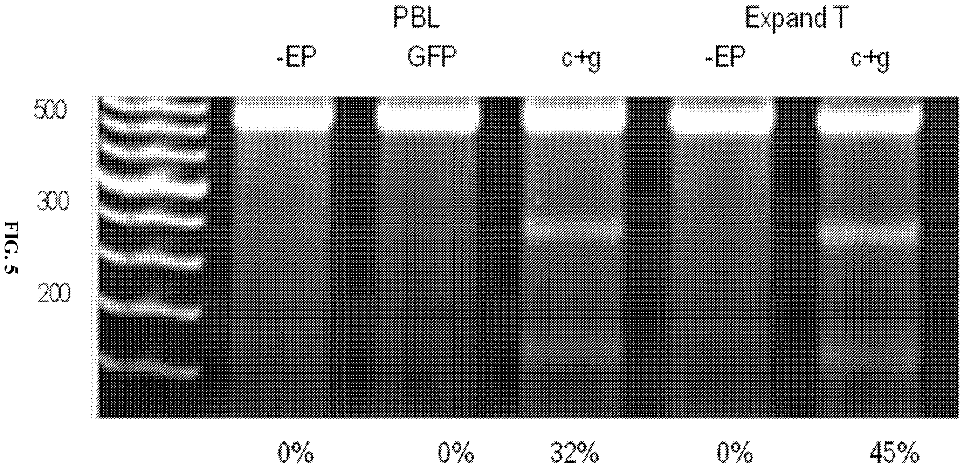

**FIG. 5****: mRNA-CRISPR transfection induced genomic DNA editing at AAVS1 site of PBL and expanded T cells**. Depicted in FIG. 5 is a comparison of resting PBL cells to expanded T cells. Cells were either not transfected (-EP), transfected with GFP-mRNA, or transfected with mRNA-CRISPR (Cas9+gRNA, c+g). Samples were loaded in the sequence of marker, -EP, GFP and c+g of PBL and -EP and c+g of expanded T cells. The cut products of a corrected AAVS-1 site are 298 and 170 basepairs, and the parental band is 468 base pairs. The editing rate was calculated as (density of digested bands)/[(density of digested bands + density of parental band). PBL and Expanded T cells electroporated with Cas9 and guide RNA exhibited 32 and 45% editing, respectively.

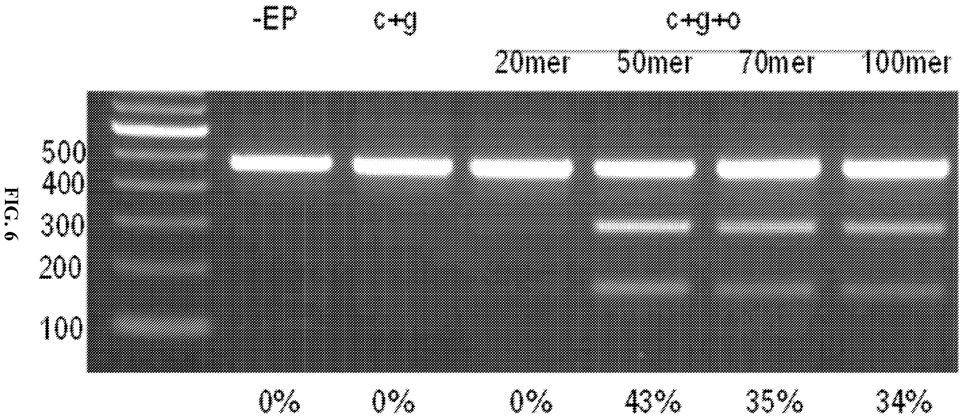

**FIG. 6****: Single-stranded-DNA-Oligo size dependent of mRNA-CRISPR transfection induced Hind III sequence integration in AAVS1 site of K562.** Cells were not transfected (-EP), transfected with mRNA-CRISPR alone (c+g), or transfected with mRNA-CRISPR plus Single-stranded-DNA-Oligo with different size as indicated. The samples were loaded in the sequence of marker, c+g, c+g+26mer, c+g+50mer, c+g+70mer and c+g+100mer. HindIII recognizing six nucleotides was into the AAVS1 site which created a HindIII digestion site. The cut products of an AAVS-1 site with integrated oligo donor sequences are 298 and 170 basepairs, and the parental band is 468 base pairs. The integration rate was calculated as (density of digested bands)/[(density of digested bands + density of parental band). The 50, 70, and 100 nucleic acid donor oligo exhibited 43, 35, and 34% integration, respectively, while the 20 nucleic acid exhibited 0% integration.

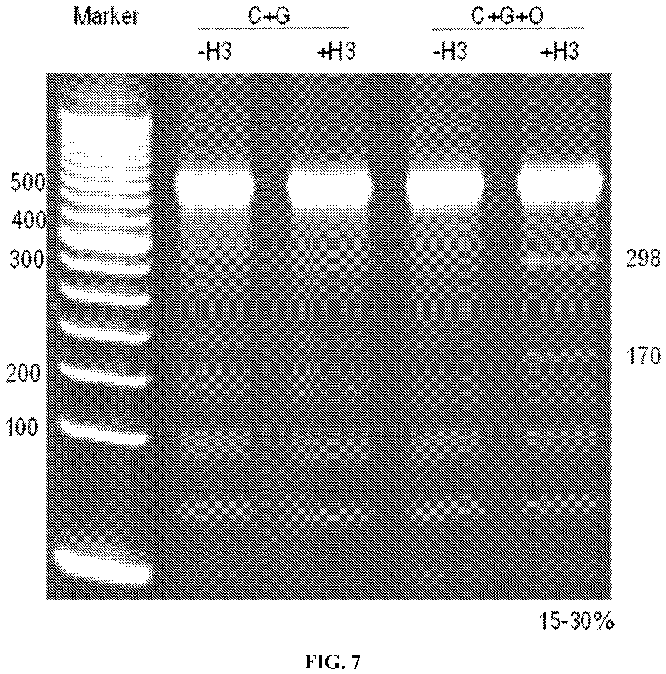

**FIG. 7****: mRNA-CRISPR oligo transfection induced Hind III sequence integration in AAVS1 site of expanded T cells.** Cells were transfected either with mRNA-CRISPR alone or with mRNA-CRISPR plus 50mer single-stranded oligo (c+g+o). The PCR amplicons were either digested (+H3) or not digested (-H3) with HidIII. The samples were loaded as follows: 1) Marker; 2) c+g-H3, ; 3) c+g+H3; 4) c+g+o-H3; 5) c+g+o+H3. The donor oligo integrated 6 nucleotides into the AAVS1 site which created a HindIII digestion site. The cut products of an AAVS-1 site with integrated oligo donor sequences are 298 and 170 basepairs, and the parental band is 468 base pairs. The integration rate was calculated as (density of digested bands)/[(density of digested bands + density of parental band). Expanded T cells transfected with donor oligo exhibited 15-30% integration.

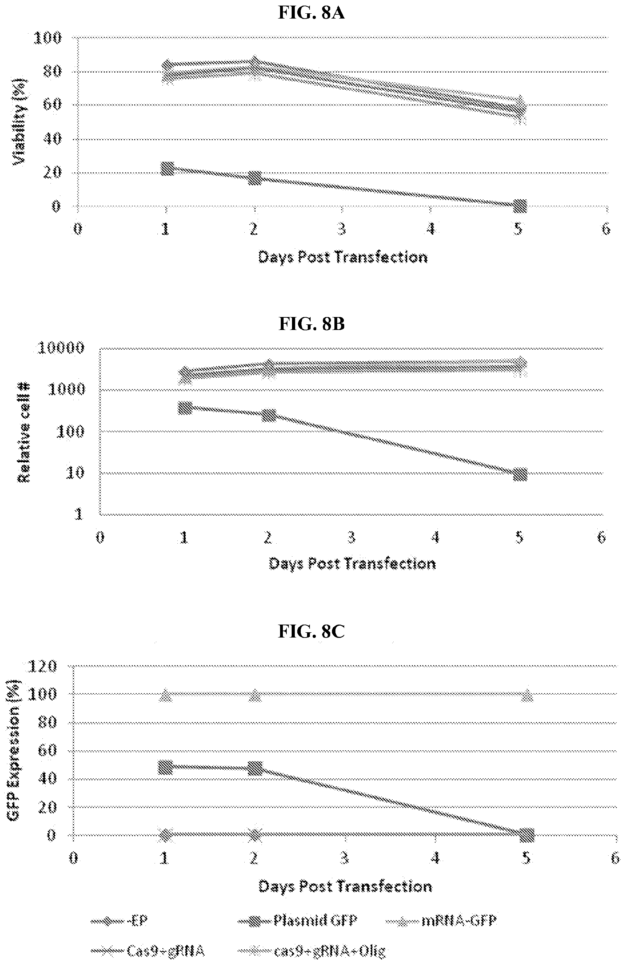

**FIG. 8A****-C: mRNA transfection by MaxCyte system has low cytotoxicity on human expanded T cells.** The viability and cell proliferation of the same expanded t cells as in Fig 7, (FIG. 8A), the proliferation of expanded T cells after transfection (FIG. 8B), and the GFP expression of expanded T cells after transfection (FIG. 8C). The data demonstrates that nucleases as mRNA with single-stranded-oligo DNA not only mediated 6 nucleotide integration (Fig 7), but also showed low cytotoxicity on expanded T cells.

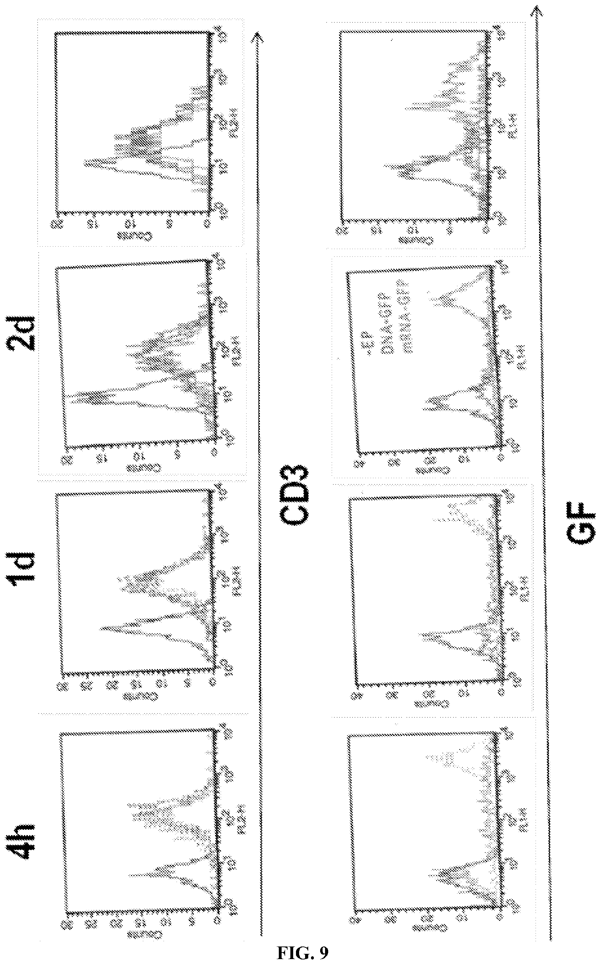

**FIG. 9****: Phenotype and GFP expression of hematopoietic stem cells (HSC).** Electroporation was done 2 days post thaw. The data indicates that transfection with mRNA is more efficient than with DNA on CD34+ HSC.

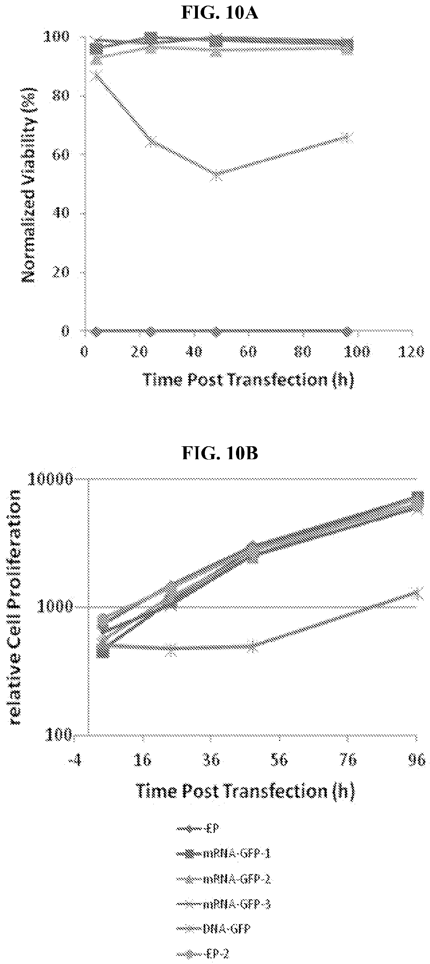

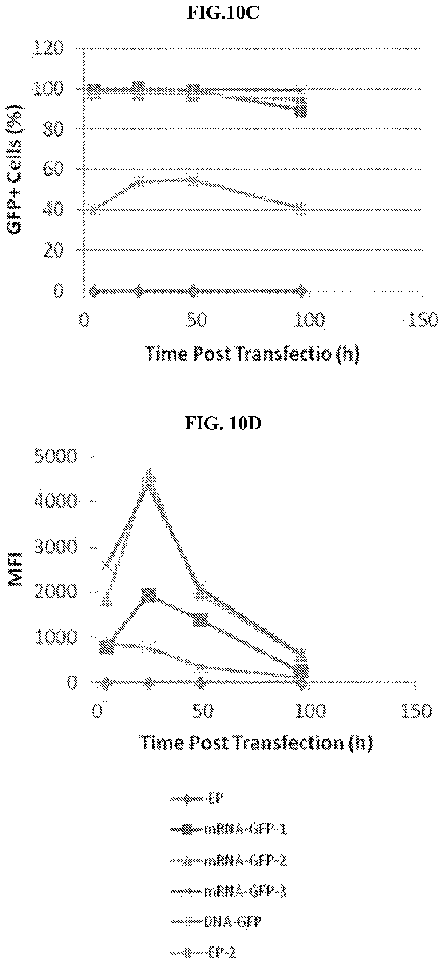

**FIG. 10A****-D: DNA-GFP transfection of HSC has much higher cytotoxicity than mRNA-GFP transfection on HSC.** HSC cells were electroporated two days after thawing. Shown is in FIG. 10 is the viability (FIG. 10A), proliferation (FIG. 10B), GFP expression (FIG. 10C), and GFP mean fluorescence intensity (MFI) (FIG. 10D) of mRNA/DNA transfected CD34+ human HSC.

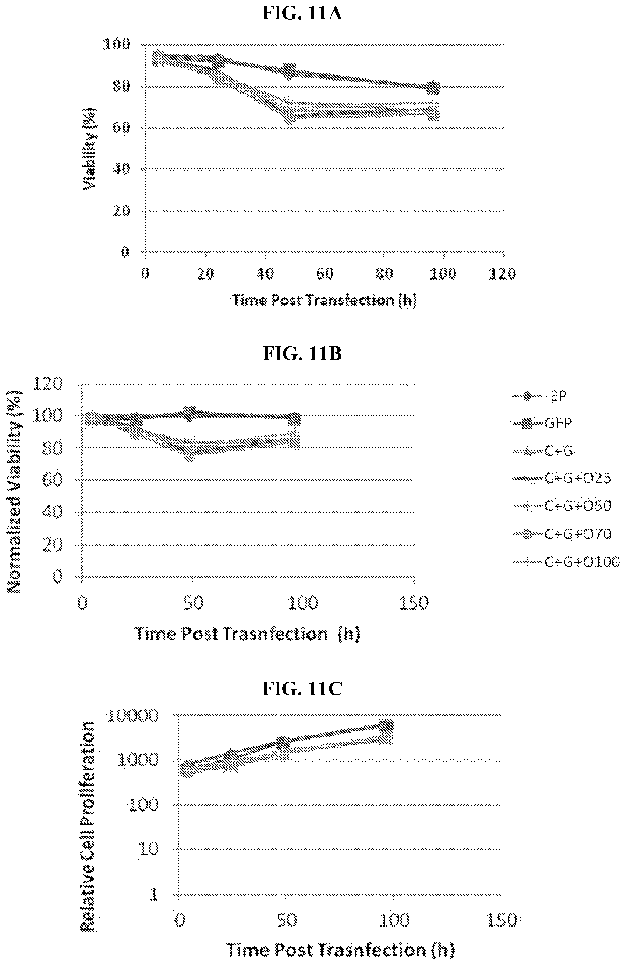

**FIG. 11A****-C: Transfection of HSC with mRNA-Cas9/gRNA plus different-sized single-stranded donor DNA oligo has low cytotoxicity**. HSC cells were electroporated two days after thawing. Shown is in FIG. 11 is the viability (FIG. 11A), normalized viability (FIG. 11B), and proliferation (FIG. 11C) of HSC transfected by mRNA-Cas9/gRNA and different-sized DNA single-stranded oligo of the indicated nucleic acid lengths.

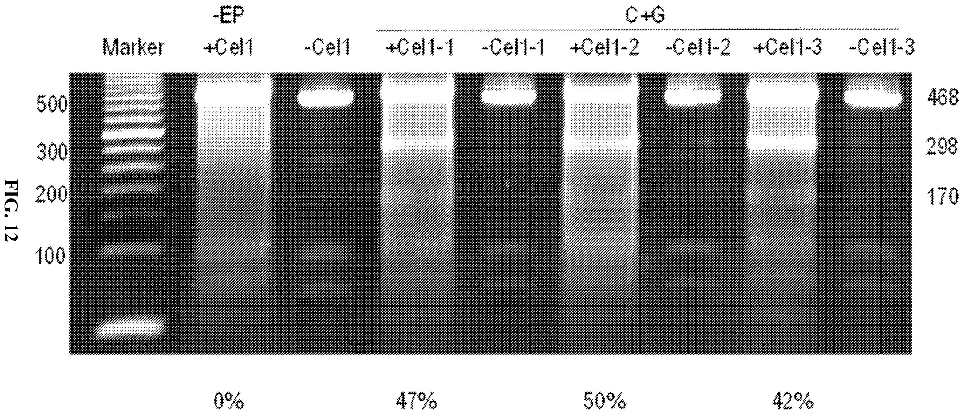

**FIG. 12****: mRNA-CRISPR transfection induced genomic DNA editing in AAVS1 site of CD34+ hematopoietic stem cells.** Cells were either not transfected (-EP), transfected with mRNA-GFP (GFP), or transfected mRNA-CRISPR with 4 repeats (C+G 1,2,3,4). The samples of the electrophoresis gel were loaded as follows: 1) Marker; 2) -EP; 3) GFP; 4) C+G-1; 5) C+G-2; 6) C+G-3; 7) C+G-4. The cut products of an edited AAVS-1 site are 298 and 170 basepairs, and the parental band is 468 base pairs. The editing rate was calculated as (density of digested bands)/[(density of digested bands + density of parental band). HSCs transfected with mRNA encoding Cas9 and guide RNA exhibited 43, 60, 54, and 52% editing in four different experiments.

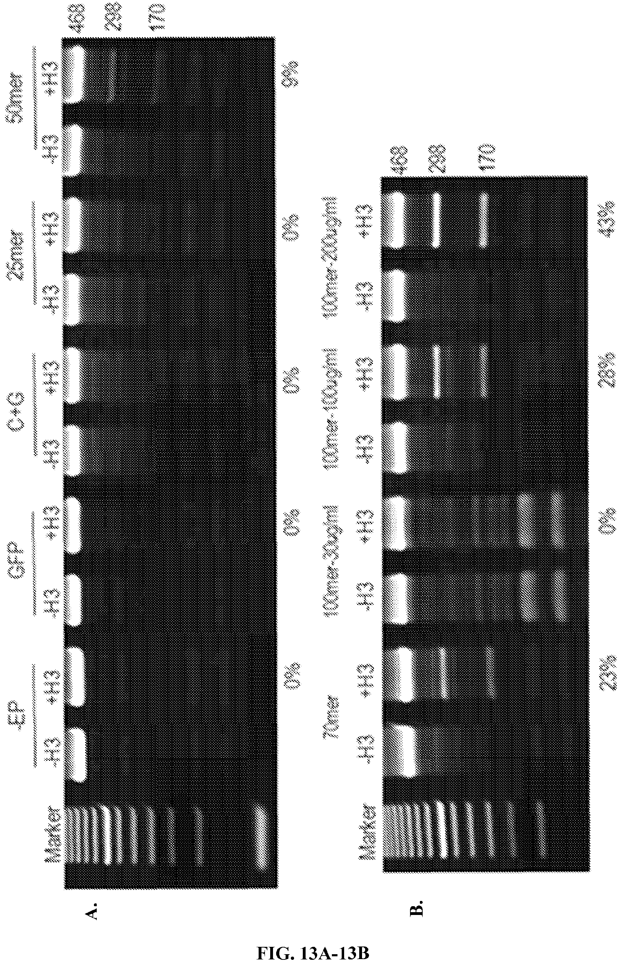

**FIG. 13A****-B: mRNA-CRISPR olig transfection induced Hind III sequence integration in AAVS1 site of CD34+ hematopietic stem cells 2d post transfection.** Cells were either not transfected (-EP), transfected with GFP-mRNA (GFP), mRNA-CRISPR (C+G) alone, or mRNA-CRISPR plus different sized-oligos (26mer, 50mer, 70mer and 100mer with indicated oligo concentrations of 100mer). The samples of the electrophoresis gel were loaded as follows: 1) Marker; 2) -EP -H3; 3) -EP +H3; 4) GFP -H3; 5) GFP +H3; 6) C+G -H3; 7) C+G +H3; 8) 26mer -H3,; 9) 26mer +H3; 10) 50mer -H3; and 11) 50mer +H3. Samples in FIG. 13B are electrophoresis gel loaded as follows: 1) Marker; 2) 70mer - H3; 3) 70mer +H3; 4) 100mer-30 µg/mL -H3; 5) 100mer-30 µg/mL +H3; 6) 100mer-100 µg/mL -H3; 7) 100mer-100 µg/mL +H3; 8) 100mer-200 µg/mL -H3; 9) 100mer-200 µg/mL +H3. The cut products of an integrated AAVS-1 site are 298 and 170 basepairs, and the parental band is 468 base pairs. The integration rate was calculated as (density of digested bands)/[(density of digested bands + density of parental band). HSCs transfected with 25mer nucleic acid DNA oligo exhibited 0% integration while HSCs transfected with a 50mer and 70mer nucleic acid oligo exhibited 9 and 23% integration, respectfully. HSCs transfected with 30 µg/mL of a 100 nucleotide oligo exhibited 0% integration at this time point (13% at 4d post transfection, data not shown) while HSCs transfected with 100 µg/mL and 200 µg/mL of the same oligo exhibited 28 and 43% integration, respectfully.

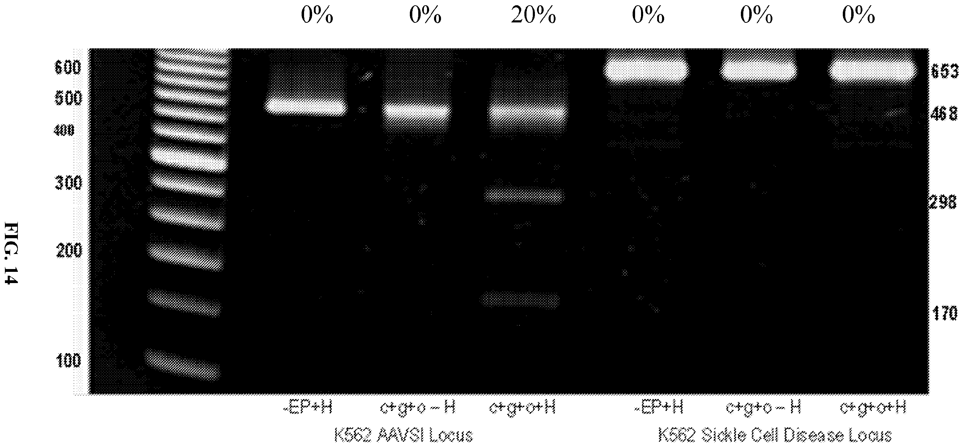

**FIG. 14****: Guide RNA provides integration specificity. An oligo with gRNA targeting AAVS1 site integrates in AAVS1, but not in the sickle cell disease (SCD) locus.** Cells were either not electroporated (-EP) or electroporated with mRNA-CRISPR plus donor oligo (c+g+o). -/+ H indicates the absence (-) or presence (+) of HindIII endonuclease. The samples of the electrophoresis gel were loaded as follows: 1) Marker; 2) -EP +H; 3) c+g+o-H; 4) c+g+o+H; 5) -EP +H; 6) c+g+o-H; 7) c+g+o+H. Lanes 2-4 represent genomic DNA from the AAVS1 locus and lanes 5-7 represent genomic DNA from SCD locus. The cut products of an integrated AAVS1 site are 298 and 170 basepairs, and the parental band is 468 base pairs. The integration rate was calculated as (density of digested bands)/[(density of digested bands + density of parental band). K562 cells transfected with DNA oligo and a AAVS1 locus-specific guide RNA integrates specifically into the AAVS1 site and not the SCD locus. The integration rate at the AAVS1 locus was 20%.

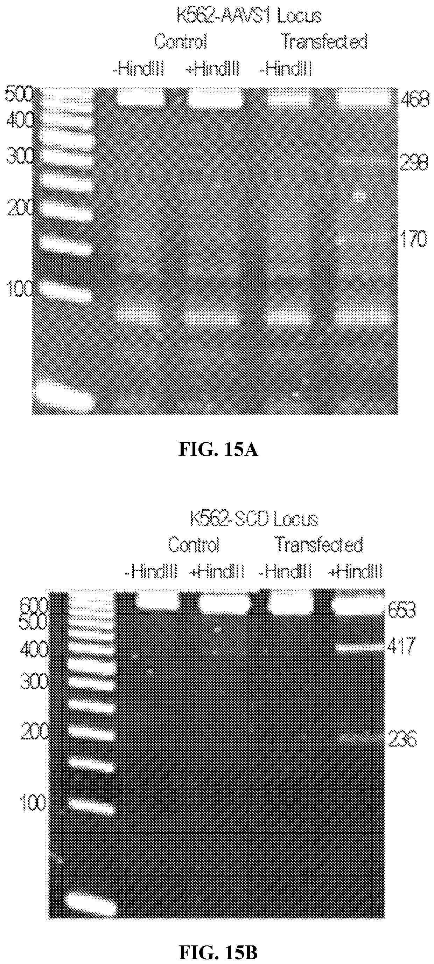

**FIG. 15A****-B: Site-specific inetegration using two guide RNAs targeting the AAVS1 (****FIG. 15A****) and SCD locus (****FIG. 15B****).** As shown in FIG. 15B, site-specific integration of donor DNA was achieved at the SCD locus. These results are further described in Example 2.

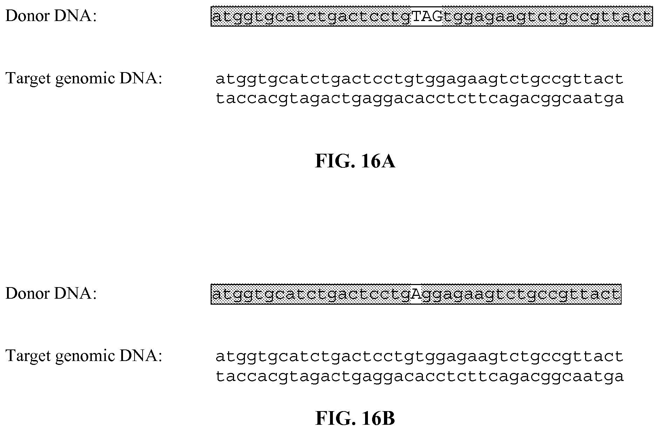

**FIG. 16A****-B: Example donor DNA oligo with sequence modification region (uppercase and not shaded) and homologous region (lower case and shaded).** FIG. 16A shows an example where a stop codon is inserted as an addition into a target genomic DNA. (SEQ ID NOs. 40 and 41) FIG. 16B shown an example where a single base is changed in the target genomic DNA. (SEQ ID NOs. 42 and 43).

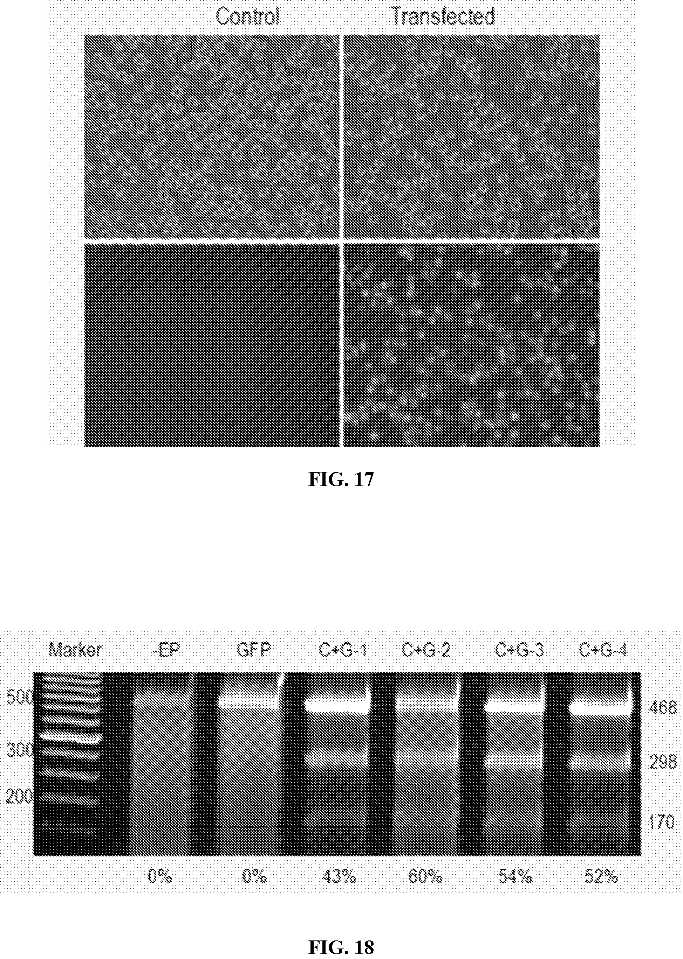

**FIG. 17****: Efficient transfection of HSC with mRNA encoding eGFP 1d post transfection.** Control cells (without transfection, left two micrographs) and the transfected cells are presented (rgiht two micrograph). Cells are viable for both control and transfected cells. The close to 100% expression of eGFP (bottom, right) demonstrates the efficient transfection efficiency with mRNA transfection.
**FIG. 18****: Electroporation mediated efficient gene editing at AAVS1 site in HSC.** HSC was transfected with cas9 (c) and gRNA (g) in mRNA formulation. Cel-1 assay was performed for the analysis of gene editing. Lane 1 is marker. Lane 2 is the control HSC (-EP). Lane 3 is GFP-mRNA transfected HSC. Lane 4 to 7 are the quadrate transfections of HSC with Cas9/gRNA.

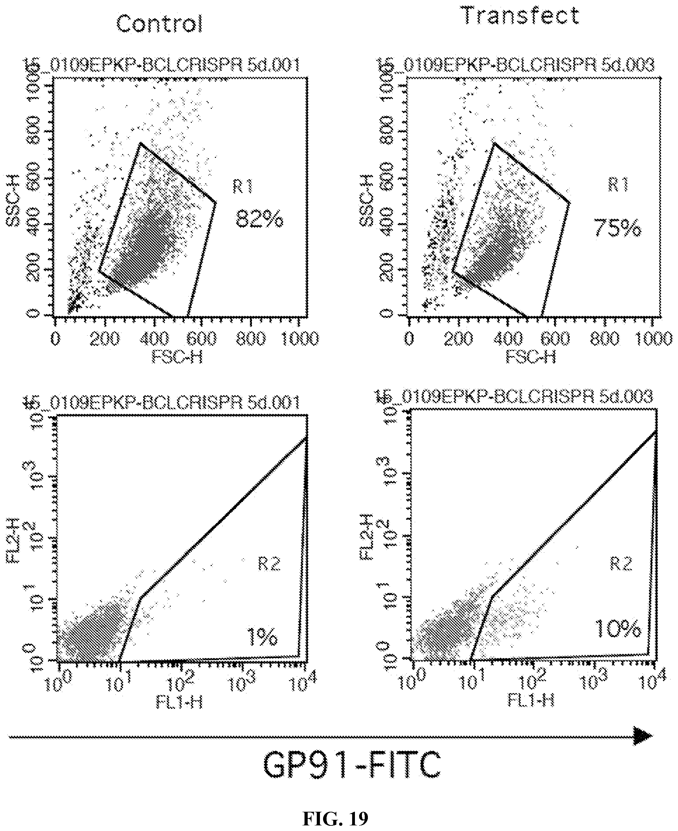

**FIG. 19****:** The most prevalent mutation (a 'hotspot') in gp91phox is the position 676C to T mutation in Exon 7. With the use of CRISPR and donor DNA single-stranded oligo to correct the T mutation back to C, from stop codon in CGD back to Arg at amino site of 226 after correction, it will restore the gp91 expression. By using EBV-transformed B cells derived from CGD patients, the cotranasfection actually restored the gp91 expression when assayed at 5d post Transfection with FITC-conjugated antibody against pg91 from 1% basal noise level (bottom left) to 10% upregulated level (bottom right). The transfection was done two days after thawing the cells.

### DESCRIPTION OF ILLUSTRATIVE EMBODIMENTS

Methods described herein use a DNA oligo and a DNA digesting agent to modify/amend a DNA sequence. It is contemplated that methods described herein provide a low toxicity and a high efficiency of incorporation of the DNA sequence modification.

### Nucleic acids

### B. Oligo

Embodiments concern the sequence modification of target genomic DNA sequences by electroporating cells with a composition comprising a DNA oligo and a DNA digesting agent. In some embodiments, the DNA oligo is single-stranded.

The term "endogenous genomic DNA" refers to the chromosomal DNA of the cell. The term "target genomic DNA sequence" refers to an endogenous genomic DNA site in which a DNA sequence modification is directed to. The DNA sequence modification may be one that changes one or more bases of the target genomic DNA sequence at one specific site or multiple specific sites. A change may include changing at least, at most, or exactly 1, 2, 3, 4, 5, 10, 15, 20, 25, 30, 35, 40 base pairs or any derivable range therein of the target genomic DNA sequence to a different at least, at most, or exactly 1, 2, 3, 4, 5, 10, 15, 20, 25, 30, 35, 40 base pairs or any derivable range therein. A deletion may be a deletion of at least, at most, or exactly 1, 2, 3, 4, 5, 10, 15, 20, 25, 30, 40, 50, 75, 100, 150, 200, 300, 400, or 500 base pairs or any range derivable therein. An addition may be the addition of at least, at most, or exactly 1, 2, 3, 4, 5, 10, 15, 20, 25, 30, 35, 40 or more base pairs or any range derivable therein. A sequence modification or amendment may be classified as a change and deletion, a change and addition, etc... if the sequence modification alters the target genomic DNA in multiple ways. In one embodiment, the sequence modification is a stop codon. In a further embodiment, the DNA sequence modification is one or more stop codons. In further embodiments, the DNA sequence modification is 1, 2, 3, 4, 5, or 10 stop codons. When the sequence modification is a stop codon, efficiency and/or reliability of gene editing may be increased.

The term "oligo" or "oligonucleotide" refers to polynucleotides such as deoxyribonucleic acid (DNA), and, where appropriate, ribonucleic acid (RNA). The term should also be understood to include, as equivalents, derivatives, variants and analogs of either RNA or DNA made from nucleotide analogs, and, as applicable to the embodiment being described, single (sense or antisense) and double-stranded polynucleotides. Deoxyribonucleotides include deoxyadenosine, deoxycytidine, deoxyguanosine, and deoxythymidine. For purposes of clarity, when referring herein to a nucleotide of a nucleic acid, which can be DNA or an RNA, the terms "adenosine", "cytidine", "guanosine", and "thymidine" are used. It is understood that if the nucleic acid is RNA, a nucleotide having a uracil base is uridine.

The terms "polynucleotide" and "oligonucleotide" are used interchangeably and refer to a polymeric form of nucleotides of any length, either deoxyribonucleotides or ribonucleotides or analogs thereof. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any embodiment of this invention that is a polynucleotide encompasses both the double-stranded form and each of two complementary single-stranded forms known or predicted to make up the double-stranded form.

The DNA oligo described herein comprises a sequence complementary to the target genomic DNA sequence and a sequence modification of the target genomic DNA sequence.

The term "complementary" as used herein refers to Watson-Crick base pairing between nucleotides and specifically refers to nucleotides hydrogen bonded to one another with thymine or uracil residues linked to adenine residues by two hydrogen bonds and cytosine and guanine residues linked by three hydrogen bonds. In general, a nucleic acid includes a nucleotide sequence described as having a "percent complementarity" to a specified second nucleotide sequence. For example, a nucleotide sequence may have 80%, 90%, or 100% complementarity to a specified second nucleotide sequence, indicating that 8 of 10, 9 of 10 or 10 of 10 nucleotides of a sequence are complementary to the specified second nucleotide sequence. For instance, the nucleotide sequence 3'-TCGA-5' is 100% complementary to the nucleotide sequence 5'-AGCT-3'. Further, the nucleotide sequence 3'-TCGA- is 100% complementary to a region of the nucleotide sequence 5'-TTAGCTGG-3'. It will be recognized by one of skill in the art that two complementary nucleotide sequences include a sense strand and an antisense strand.

In certain embodiments, the oligo comprises at least about 10, 12, 14, 16, 18, 20, 22, 24, 26, 28, 30, 32, 34, 36, 38, 40, 42, 44, 46, 48, or 50 nucleic acids of sequence that is complementary to the target genomic DNA sequence. In specific embodiments, the oligo comprises at least about 20 nucleic acids of sequence that are complementary to the genomic DNA sequence. In this context, the term "complimentary sequence" refers to sequence that exactly matches the sequence of the genomic DNA. The complimentary sequence may be in a region that is on the 5' end of the DNA sequence modification and in a region that is on the 3' end of a DNA sequence modification. By way of illustrative example, when the oligo comprises at least 20 nucleic acids of complimentary sequences, the oligo may comprise, for example, 10 nucleic acids of complimentary sequence on each side of the sequence modification. Similarly, an oligo comprising 10 nucleic acids of complimentary sequences may comprise, for example, 5 nucleic acids of complimentary sequence on each side of the sequence modification.

The DNA oligo may be from about 10, 20, 25, 30, 35, 40, 50, 60, 70, 80, 90, 100, 150, 200, 250, 300, 350, 400, 450, 500, 550, or 600 nucleic acids to about 100, 125, 150, 175, 200, 225, 250, 275, 300, 325, 350, 375, 400, 425, 450, 475, 500 nucleic acids in length, or any derivable range thereof. In certain embodiments, the oligo is more than 20 nucleic acids, or more than 21, 22, 23, 24, 25, 30, or 40 nucleic acids. In specific embodiments, the oligo is from about 30 to 300 nucleic acids, from about 25 to about 200 nucleic acids, from about 25 to about 150 nucleic acids, from about 25 to about 100 nucleic acids, or from about 40 to about 100 nucleic acids.

The concentration of the oligo during the electroporation procedure may be the final concentration of the oligo in the electroporation chamber and/or sample container. The oligo concentration may be from about 10, 20, 30, 50, 75, 100, 150, 200, 250, 300 to about 350, 400, 500, 1000, 1500, 2000, 3000, 4000, or 5000 µg/mL or any range derivable therein. In certain embodiments, the concentration of the oligo is at least 30 µg/mL. In further embodiments, the concentration of the oligo is at least,at most, or exactly 1 0, 15, 20, 25, 30, 35, 40, 45, 50, 55, 60, 65, 70, 75, 80, 85, 90, 95, 100, 150, or 200 µg/mL or any derivable range therein.

### C. DNA digesting agent

The present invention provides methods for modifying a target genomic DNA sequence by transfecting the cells by electroporation with a DNA oligo and a DNA digesting agent. The term "DNA digesting agent" refers to an agent that is capable of cleaving bonds (i.e. phosphodiester bonds) between the nucleotide subunits of nucleic acids. In a specific embodiment, the DNA digesting agent is encoded on RNA. It is contemplated that providing the DNA digesting agent on RNA may do one or more of improve viability of the cells after transfection and increase efficiency of sequence modification. In other embodiments, the DNA digesting agent is a protein, an enzyme, or a small molecule mimic that has enzymatic activity.

In one embodiment, the DNA digesting agent is a transposase. For example, a synthetic DNA transposon (e.g. "Sleeping Beauty" transposon system) designed to introduce precisely defined DNA sequences into the chromosome of vertebrate animals can be used. The Sleeping Beauty transposon system is composed of a Sleeping Beauty (SB) transposase and a transposon that was designed to insert specific sequences of DNA into genomes of vertebrate animals. DNA transposons translocate from one DNA site to another in a simple, cut-and-paste manner. Transposition is a precise process in which a defined DNA segment is excised from one DNA molecule and moved to another site in the same or different DNA molecule or genome.

As do all other Tc1/mariner-type transposases, SB transposase inserts a transposon into a TA dinucleotide base pair in a recipient DNA sequence. The insertion site can be elsewhere in the same DNA molecule, or in another DNA molecule (or chromosome). In mammalian genomes, including humans, there are approximately 200 million TA sites. The TA insertion site is duplicated in the process of transposon integration. This duplication of the TA sequence is a hallmark of transposition and used to ascertain the mechanism in some experiments. The transposase can be encoded either within the transposon or the transposase can be supplied by another source, in which case the transposon becomes a non-autonomous element. Non-autonomous transposons are most useful as genetic tools because after insertion they cannot independently continue to excise and re-insert. All of the DNA transposons identified in the human genome and other mammalian genomes are non-autonomous because even though they contain transposase genes, the genes are non-functional and unable to generate a transposase that can mobilize the transposon.

In a further embodiment, the DNA digesting agent is an integrase. For example, The phiC31 integrase is a sequence-specific recombinase encoded within the genome of the bacteriophage phiC31. The phiC31 integrase mediates recombination between two 34 base pair sequences termed attachment sites (att), one found in the phage and the other in the bacterial host. This serine integrase has been show to function efficiently in many different cell types including mammalian cells. In the presence of phiC31 integrase, an attB-containing donor plasmid can be unidirectional integrated into a target genome through recombination at sites with sequence similarity to the native attP site (termed pseudo-attP sites). phiC31 integrase can integrate a plasmid of any size, as a single copy, and requires no cofactors. The integrated transgenes are stably expressed and heritable.

In a specific embodiment, the DNA digesting agent is a nuclease. Nucleases are enzymes that hydrolyze nucleic acids. Nucleases may be classified as endonucleases or exonucleases. An endonuclease is any of a group enzymes that catalyze the hydrolysis of bonds between nucleic acids in the interior of a DNA or RNA molecule. An exonuclease is any of a group of enzymes that catalyze the hydrolysis of single nucleotides from the end of a DNA or RNA chain. Nucleases may also be classified based on whether they specifically digest DNA or RNA. A nuclease that specifically catalyzes the hydrolysis of DNA may be referred to as a deoxyribonuclease or DNase, whereas a nuclease that specifically catalyses the hydrolysis of RNA may be referred to as a ribonuclease or an RNase. Some nucleases are specific to either single-stranded or double-stranded nucleic acid sequences. Some enzymes have both exonuclease and endonuclease properties. In addition, some enzymes are able to digest both DNA and RNA sequences. The term "nuclease" is used herein to generally refer to any enzyme that hydrolyzes nucleic acid sequences.

Optimal reaction conditions vary among the different nucleases. The factors that should be considered include temperature, pH, enzyme cofactors, salt composition, ionic strength, and stabilizers. Suppliers of commercially available nucleases (e.g., Promega Corp.; New England Biolabs, Inc.) provide information as to the optimal conditions for each enzyme. Most nucleases are used between pH 7.2 and pH 8.5 as measured at the temperature of incubation. In addition, most nucleases show maximum activity at 37° C.; however, a few enzymes require higher or lower temperatures for optimal activity (e.g., Taq I, 65° C; Sma I, 25° C). DNA concentration can also be a factor as a high DNA concentration can reduce enzyme activity, and DNA concentrations that are too dilute can fall below the Kₘ of the enzyme and also affect enzyme activity.

Non-limiting examples of nucleases include, DNase I, Benzonase, Exonuclease I, Exonuclease III, Mung Bean Nuclease, Nuclease BAL 31, RNase I, S1 Nuclease, Lambda Exonuclease, RecJ, and T7 exonuclease. DNase I is an endonuclease that nonspecifically cleaves DNA to release di-, tri- and oligonucleotide products with 5'-phosphorylated and 3'-hydroxylated ends. DNase I acts on single- and double-stranded DNA, chromatin, and RNA:DNA hybrids. Exonuclease I catalyzes the removal of nucleotides from single-stranded DNA in the 3' to 5' direction. Exonuclease III catalyzes the stepwise removal of mononucleotides from 3'-hydroxyl termini of duplex DNA. Exonuclease III also acts at nicks in duplex DNA to produce single-strand gaps. Single-stranded DNA is resistant to Exonuclease III. Mung Bean Nuclease degrades single-stranded extensions from the ends of DNA. Mung Bean Nuclease is also an RNA endonuclease. Nuclease BAL 31 degrades both 3' and 5' termini of duplex DNA. Nuclease BAL 31 is also a highly specific single-stranded endonuclease that cleaves at nicks, gaps, and single-stranded regions of duplex DNA and RNA. RNase I is a single strand specific RNA endonuclease that will cleave at all RNA dinucleotide. S1 Nuclease degrades single-stranded DNA and RNA endonucleolytically to yield 5'-phosphoryl-terminated products. Double-stranded nucleic acids (DNA:DNA, DNA:RNA or RNA:RNA) are resistant to S1 nuclease degradation except with extremely high concentrations of enzyme. Lambda Exonuclease catalyzes the removal of 5' mononucleotides from duplex DNA. Its preferred substrate is 5'-phosphorylated double stranded DNA, although Lambda Exonuclease will also degrade single-stranded and non-phosphorylated substrates at a greatly reduced rate. Lambda Exonuclease is unable to initiate DNA digestion at nicks or gaps, RecJ is a single-stranded DNA specific exonuclease that catalyzes the removal of deoxy-nucleotide monophosphates from DNA in the 5' to 3' direction. T7 exonuclease catalyzes the removal of 5' mononucleotides from duplex DNA. T7 Exonuclease catalyzes nucleotide removal from the 5' termini or at gaps and nicks of double-stranded DNA.

Restriction endonucleases are another example of nucleases that may be used in connection with the methods of the present invention. Non-limiting examples of restriction endonucleases and their recognition sequences are provided in Table 1.

**Table 1. Recognition Sequences for Restriction Endonucleases.**

| **ENZYME** | **RECOGNITION SEQUENCE** | **SEQ ID NO.** | **ENZYME** | **RECOGNITION SEQUENCE** | **SEQ ID NO.** |
|---|---|---|---|---|---|
| AatII | GACGTC | | Fnu4H I | GCNGC | |
| Acc65 I | GGTACC | | Fok I | GGATG | |
| Acc I | GTMKAC | | Fse I | GGCCGGCC | |
| Aci I | CCGC | | Fsp I | TGCGCA | |
| Acl I | AACGTT | | Hae II | RGCGCY | |
| Afe I | AGCGCT | | Hae III | GGCC | |
| Afl II | CTTAAG | | Hga I | GACGC | |
| Afl III | ACRYGT | | Hha I | GCGC | |
| Age I | ACCGGT | | Hine II | GTYRAC | |
| Ahd I | GACNNNNNGTC | 1 | Hind III | AAGCTT | |
| Alu I | AGCT | | Hinf I | GANTC | |
| Alw I | GGATC | | HinP1 I | GCGC | |
| AlwN I | CAGNNNCTG | | Hpa I | GTTAAC | |
| Apa I | GGGCCC | | Hpa II | CCGG | |
| ApaL I | GTGCAC | | Hph I | GGTGA | |
| Apo I | RAATTY | | Kas I | GGCGCC | |
| Asc I | GGCGCGCC | | Kpn I | GGTACC | |
| Ase I | ATTAAT | | Mbo I | GATC | |
| Ava I | CYCGRG | | Mbo II | GAAGA | |
| Ava II | GGWCC | | Mfe I | CAATTG | |
| Avr II | CCTAGG | | Mlu I | ACGCGT | |
| Bae I | NACNNNNGTAPyCN | 2 | Mly I | GAGTCNNNNN | 11 |
| BamH I | GGATCC | | Mnl I | CCTC | |
| Ban I | GGYRCC | | Msc I | TGGCCA | |
| Ban II | GRGCYC | | Mse I | TTAA | |
| Bbs I | GAAGAC | | Msl I | CAYNNNNRTG | 12 |
| Bbv I | GCAGC | | MspA1 I | CMGCKG | |
| BbvC I | CCTCAGC | | Msp I | CCGG | |
| Bcg I | CGANNNNNNTGC | 3 | Mwo I | GCNNNNNNNGC | 13 |
| BciV I | GTATCC | | Nae I | GCCGGC | |
| Bcl I | TGATCA | | Nar I | GGCGCC | |
| Bfa I | CTAG | | Nei I | CCSGG | |
| Bgl I | GCCNNNNNGGC | 4 | Neo I | CCATGG | |
| Bgl II | AGATCT | | Nde I | CATATG | |
| Blp I | GCTNAGC | | NgoMI V | GCCGGC | |
| Bmr I | ACTGGG | | Nhe I | GCTAGC | |
| Bpm I | CTGGAG | | Nla III | CATG | |
| BsaA I | YACGTR | | Nla IV | GGNNCC | |
| BsaB I | GATNNNNATC | 5 | Not I | GCGGCCGC | |
| BsaH I | GRCGYC | | Nru I | TCGCGA | |
| Bsa I | GGTCTC | | Nsi I | ATGCAT | |
| BsaJ I | CCNNGG | | Nsp I | RCATGY | |
| BsaW I | WCCGGW | | Pac I | TTAATTAA | |
| BseR I | GAGGAG | | PaeR7 I | CTCGAG | |
| Bsg I | GTGCAG | | Pci I | ACATGT | |
| BsiE I | CGRYCG | | PflF I | GACNNNGTC | |
| BsiHKA I | GWGCWC | | PflM I | CCANNNNNTGG | 14 |
| BsiW I | CGTACG | | PleI | GAGTC | |
| Bsl I | CCNNNNNNNGG | 6 | Pme I | GTTTAAAC | |
| BsmA I | GTCTC | | Pml I | CACGTG | |
| BsmB I | CGTCTC | | PpuM I | RGGWCCY | |
| BsmF I | GGGAC | | PshA I | GACNNNNGTC | 15 |
| Bsm I | GAATGC | | Psi I | TTATAA | |
| BsoB I | CYCGRG | | PspG I | CCWGG | |
| Bsp1286 I | GDGCHC | | PspOM I | GGGCCC | |
| BspD I | ATCGAT | | Pst I | CTGCAG | |
| BspE I | TCCGGA | | Pvu I | CGATCG | |
| BspH I | TCATGA | | Pvu II | CAGCTG | |
| BspM I | ACCTGC | | Rsa I | GTAC | |
| BsrB I | CCGCTC | | Rsr II | CGGWCCG | |
| BsrD I | GCAATG | | Sac I | GAGCTC | |
| BsrF I | RCCGGY | | Sac II | CCGCGG | |
| BsrG I | TGTACA | | Sail | GTCGAC | |
| Bsr I | ACTGG | | Sap I | GCTCTTC | |
| BssH II | GCGCGC | | Sau3A I | GATC | |
| BssK I | CCNGG | | Sau96 I | GGNCC | |
| Bst4C I | ACNGT | | Sbf I | CCTGCAGG | |
| BssS I | CACGAG | | Sea I | AGTACT | |
| BstAP I | GCANNNNNTGC | 7 | SerF I | CCNGG | |
| BstB I | TTCGAA | | SexA I | ACCWGGT | |
| BstE II | GGTNACC | | SfaN I | GCATC | |
| BstF5 I | GGATGNN | | Sfe I | CTRYAG | |
| BstN I | CCWGG | | Sfi I | GGCCNNNNNGGCC | 16 |
| BstU I | CGCG | | Sfo I | GGCGCC | |
| BstX I | CCANNNNNNTGG | 8 | SgrA I | CRCCGGYG | |
| BstY I | RGATCY | | Sma I | CCCGGG | |
| BstZ17 I | GTATAC | | Sml I | CTYRAG | |
| Bsu36 I | CCTNAGG | | SnaB I | TACGTA | |
| Btg I | CCPuPyGG | | Spe I | ACTAGT | |
| Btr I | CACGTG | | Sph I | GCATGC | |
| Cac8 I | GCNNGC | | Ssp I | AATATT | |
| Cla I | ATCGAT | | Stu I | AGGCCT | |
| Ode I | CTNAG | | Sty I | CCWWGG | |
| Dpn I | GATC | | Swa I | ATTTAAAT | |
| Dpn II | GATC | | Taq I | TCGA | |
| Dra I | TTTAAA | | Tfi I | GAWTC | |
| Dra III | CACNNNGTG | | Tli I | CTCGAG | |
| Drd I | GACNNNNNNGTC | 9 | Tse I | GCWGC | |
| Eae I | YGGCCR | | Tsp45 I | GTSAC | |
| Eag I | CGGCCG | | Tsp509 I | AATT | |
| Far I | CTCTTC | | TspR I | CAGTG | |
| Eci I | GGCGGA | | Tth111 I | GACNNNGTC | |
| EcoN I | CCTNNNNNAGG | 10 | Xba I | TCTAGA | |
| EcoO109 I | RGGNCCY | | Xcm I | CCANNNNNNNNNTGG | 17 |
| EcoR I | GAATTC | | Xho I | CTCGAG | |
| EcoR V | GATATC | | Xma I | CCCGGG | |
| Fau I | CCCGCNNNN | | Xmn I | GAANNNNTTC | 18 |

| | | | | | |
|---|---|---|---|---|---|
| Where R = A or G, K = G or T, S = G or C, Y = C or T, M = A or C, W = A or T, B = not A (C, G or T), H = not G (A, C or T), D = not C (A, G or T), V = not T (A, C or G), and N = any nucleotide. | | | | | |

Those of ordinary skill in the art will be able to select an appropriate nuclease depending on the characteristics of the target genomic sequence and DNA oligo. In one embodiment, the nuclease is a site-specific nuclease. In a related embodiment, the nuclease has a recognition sequence of at least 8, at least 10, at least 12, at least 14, at least 16, at least 18, at least 20, or at least 25 base pairs. It is contemplated that transfecting an RNA encoding a nuclease with a recognition sequence of more than 8, 10, 12, 14, 16, 18, 20, or 25 base pairs will be less toxic to the cell. Furthermore, providing the nuclease as an RNA may also reduce toxicity to the cell.

In one embodiment, the RNA encoding a site-specific nuclease encodes a Cas nuclease. In a related embodiment, the Cas nuclease is Cas9. In a further embodiment, the nuclease is cas9 and the composition further comprises a guide RNA. Another example of a sequence-specific nuclease system that can be used with the methods and compositions described herein includes the Cas9/CRISPR system (Wiedenheft, B. et al. Nature 482, 331-338 (2012); Jinek, M. et al. Science 337, 816-821 (2012); Mali, P. et al. Science 339, 823-826 (2013); Cong, L. et al. Science 339, 819-823 (2013)). The Cas9/CRISPR (Clustered Regularly interspaced Short Palindromic Repeats) system exploits RNA-guided DNA-binding and sequence-specific cleavage of target DNA. The guide RNA/Cas9 combination confers site specificity to the nuclease. A guide RNA (gRNA) contains about 20 nucleotides that are complementary to a target genomic DNA sequence upstream of a genomic PAM (protospacer adjacent motifs) site (NNG) and a constant RNA scaffold region. The Cas (CRISPR-associated)9 protein binds to the gRNA and the target DNA to which the gRNA binds and introduces a double-strand break in a defined location upstream of the PAM site. Cas9 harbors two independent nuclease domains homologous to HNH and RuvC endonucleases, and by mutating either of the two domains, the Cas9 protein can be converted to a nickase that introduces single-strand breaks (Cong, L. et al. Science 339, 819-823 (2013)). It is specifically contemplated that the inventive methods and compositions can be used with the single- or double-strand-inducing version of Cas9, as well as with other RNA-guided DNA nucleases, such as other bacterial Cas9-like systems. The sequence-specific nuclease of the methods and compositions described herein can be engineered, chimeric, or isolated from an organism. The sequence-specific nuclease can be introduced into the cell in form of an RNA encoding the sequence-specific nuclease, such as an mRNA.

In one embodiment, the RNA encoding a site-specific nuclease encodes a zinc finger nuclease. Zinc finger nucleases generally comprise a DNA binding domain (i.e., zinc finger) and a cutting domain (i.e., nuclease). Zinc finger binding domains may be engineered to recognize and bind to any nucleic acid sequence of choice. See, for example, Beerli et al. (2002) Nat. Biotechnol. 20:135-141 ; Pabo et al. (2001 ) Ann. Rev. Biochem. 70:313-340; Isalan et al. (2001 ) Nat. Biotechnol. 19:656-660; Segal et al. (2001 ) Curr. Opin. Biotechnol. 12:632-637; Choo et al. (2000) Curr. Opin. Struct. Biol. 10:41 1 -416; Zhang et al. (2000) J. Biol. Chem. 275(43):33850-33860; Doyon et al. (2008) Nat. Biotechnol. 26:702-708; and Santiago et al. (2008) Proc. Natl. Acad. Sci. USA 105:5809-5814. An engineered zinc finger binding domain may have a novel binding specificity compared to a naturally-occurring zinc finger protein. Engineering methods include, but are not limited to, rational design and various types of selection. Rational design includes, for example, using databases comprising doublet, triplet, and/or quadruplet nucleotide sequences and individual zinc finger amino acid sequences, in which each doublet, triplet or quadruplet nucleotide sequence is associated with one or more amino acid sequences of zinc fingers which bind the particular triplet or quadruplet sequence. See, for example, U.S. Pat. Nos. 6,453,242 and 6,534,261. As an example, the algorithm of described in US patent 6,453,242 may be used to design a zinc finger binding domain to target a preselected sequence.

Alternative methods, such as rational design using a nondegenerate recognition code table may also be used to design a zinc finger binding domain to target a specific sequence (Sera et al. (2002) Biochemistry 41 :7074-7081). Publically available web-based tools for identifying potential target sites in DNA sequences and designing zinc finger binding domains may be found at http://www.zincfingertools.org and http://bindr.gdcb.iastate.edu/ZiFiT/, respectively (Mandell et al. (2006) Nuc. Acid Res. 34:W516-W523; Sander et al. (2007) Nuc. Acid Res. 35:W599-W605).

A zinc finger binding domain may be designed to recognize and bind a DNA sequence ranging from about 3 nucleotides to about 21 nucleotides in length, or preferably from about 9 to about 18 nucleotides in length. In general, the zinc finger binding domains comprise at least three zinc finger recognition regions (i.e., zinc fingers). In one embodiment, the zinc finger binding domain may comprise four zinc finger recognition regions. In another embodiment, the zinc finger binding domain may comprise five zinc finger recognition regions. In still another embodiment, the zinc finger binding domain may comprise six zinc finger recognition regions. A zinc finger binding domain may be designed to bind to any suitable target DNA sequence. See for example, U.S. Pat. Nos. 6,607,882; 6,534,261 and 6,453,242.

Exemplary methods of selecting a zinc finger recognition region may include phage display and two-hybrid systems, and are disclosed in U.S. Pat. Nos. 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237 In addition, enhancement of binding specificity for zinc finger binding domains has been described, for example, in WO 02/077227.

Zinc finger binding domains and methods for design and construction of fusion proteins (and polynucleotides encoding same) are known to those of skill in the art and are described in detail in U.S. Patent Application Publication Nos. 20050064474 and 20060188987 Zinc finger recognition regions and/or multi-fingered zinc finger proteins may be linked together using suitable linker sequences, including for example, linkers of five or more amino acids in length. See, U.S. Pat. Nos. 6,479,626; 6,903,185; and 7,153,94, for non- limiting examples of linker sequences of six or more amino acids in length. The zinc finger binding domain described herein may include a combination of suitable linkers between the individual zinc fingers of the protein.

In some embodiments, the zinc finger nuclease may further comprise a nuclear localization signal or sequence (NLS). A NLS is an amino acid sequence which facilitates targeting the zinc finger nuclease protein into the nucleus to introduce a double stranded break at the target sequence in the chromosome. Nuclear localization signals are known in the art. See, for example, Makkerh et al. (1996) Current Biology 6:1025-1027.

A zinc finger nuclease also includes a cleavage domain. The cleavage domain portion of the zinc finger nuclease may be obtained from any endonuclease or exonuclease. Non-limiting examples of endonucleases from which a cleavage domain may be derived include, but are not limited to, restriction endonucleases and homing endonucleases. See, for example, 2002-2003 Catalog, New England Biolabs, Beverly, Mass.; and Belfort et al. (1997) Nucleic Acids Res. 25:3379-3388 or www.neb.com. Additional enzymes that cleave DNA are known (e.g., S1 Nuclease; mung bean nuclease; pancreatic DNase I; micrococcal nuclease; yeast HO endonuclease). See also Linn et al. (eds.) Nucleases, Cold Spring Harbor Laboratory Press, 1993. One or more of these enzymes (or functional fragments thereof) may be used as a source of cleavage domains.

A cleavage domain also may be derived from an enzyme or portion thereof, as described above, that requires dimerization for cleavage activity. Two zinc finger nucleases may be required for cleavage, as each nuclease comprises a monomer of the active enzyme dimer. Alternatively, a single zinc finger nuclease may comprise both monomers to create an active enzyme dimer. As used herein, an "active enzyme dimer" is an enzyme dimer capable of cleaving a nucleic acid molecule. The two cleavage monomers may be derived from the same endonuclease (or functional fragments thereof), or each monomer may be derived from a different endonuclease (or functional fragments thereof).

When two cleavage monomers are used to form an active enzyme dimer, the recognition sites for the two zinc finger nucleases are preferably disposed such that binding of the two zinc finger nucleases to their respective recognition sites places the cleavage monomers in a spatial orientation to each other that allows the cleavage monomers to form an active enzyme dimer, e.g., by dimerizing. As a result, the near edges of the recognition sites may be separated by about 5 to about 18 nucleotides. For instance, the near edges may be separated by about 5, 6, 7, 8, 9, 10, 1 1 , 12, 13, 14, 15, 16, 17 or 18 nucleotides. It will however be understood that any integral number of nucleotides or nucleotide pairs may intervene between two recognition sites (e.g., from about 2 to about 50 nucleotide pairs or more). The near edges of the recognition sites of the zinc finger nucleases, such as for example those described in detail herein, may be separated by 6 nucleotides. In general, the site of cleavage lies between the recognition sites.

Restriction endonucleases (restriction enzymes) are present in many species and are capable of sequence-specific binding to DNA (at a recognition site), and cleaving DNA at or near the site of binding. Certain restriction enzymes (e.g., Type IIS) cleave DNA at sites removed from the recognition site and have separable binding and cleavage domains. For example, the Type IIS enzyme Fokl catalyzes double-stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91 :883-887; Kim et al. (1994b) J. Biol. Chem. 269:31 , 978-31 , 982. Thus, a zinc finger nuclease may comprise the cleavage domain from at least one Type IIS restriction enzyme and one or more zinc finger binding domains, which may or may not be engineered. Exemplary Type IIS restriction enzymes are described for example in International Publication WO 07/014,275 Additional restriction enzymes also contain separable binding and cleavage domains, and these also are contemplated by the present disclosure. See, for example, Roberts et al. (2003) Nucleic Acids Res. 31 :418-420.

In another embodiment, the targeting endonuclease may be a meganuclease. Meganucleases are endodeoxyribonucleases characterized by a large recognition site, i.e., the recognition site generally ranges from about 12 base pairs to about 40 base pairs. As a consequence of this requirement, the recognition site generally occurs only once in any given genome. Naturally-occurring meganucleases recognize 15-40 base-pair cleavage sites and are commonly grouped into four families: the LAGLIDADG family, the GIY-YIG family, the His-Cyst box family and the HNH family. Meganucleases can be targeted to specific chromosomal sequence by modifying their recognition sequence using techniques well known to those skilled in the art.

In a further embodiment, the targeting endonuclease may be a transcription activator-like effector (TALE) nuclease. TALEs are transcription factors from the plant pathogen Xanthomonas that can be readily engineered to bind new DNA targets. TALEs or truncated versions thereof may be linked to the catalytic domain of endonucleases such as Fokl to create targeting endonuclease called TALE nucleases or TALENs.

In still another embodiment, the targeting endonuclease may be a site-specific nuclease. In particular, the site-specific nuclease may be a "rare-cutter' endonuclease whose recognition sequence occurs rarely in a genome. Preferably, the recognition sequence of the site-specific nuclease occurs only once in a genome.

In yet another embodiment, the targeting endonuclease may be an artificial targeted DNA double strand break inducing agent (also called an artificial restriction DNA cutter). For example, the artificial targeted DNA double strand break inducing agent may comprise a metal/chelator complex that cleaves DNA and at least one oligonucleotide that is complementary to the targeted cleavage site. The artificial targeted DNA double strand break inducing agent, therefore, does not contain any protein, The metal of the metal/chelator complex may be cerium, cadmium, cobalt, chromium, copper, iron, magnesium, manganese, zinc, and the like. The chelator of the metal/chelator complex may be EDTA, EGTA, BAPTA, and so forth. In a preferred embodiment, the metal/chelator complex may be Ce(IV)/EGTA. In another preferred embodiment, the artificial targeted DNA double strand break inducing agent may comprise a complex of Ce(IV)/EGTA and two strands of pseudo-complementary peptide nucleic acids (PNAs) (Katada et al., Current Gene Therapy, 201 1 , 1 1 (1 ):38-45).

In a further embodiment, the nuclease may be a homing nuclease. Homing endonucleases include 1-5'cel, l-Ceul, l-Pspl, Vl-Sce, l-SceTV, I- Csml, l-Panl, l-Scell, l-Ppol, l-Scelll, l-Crel, l-Tevl, 1-Tev and I-7evIII. Their recognition sequences are known. See also U.S. Patent No. 5,420,032; U.S. Patent No. 6,833,252; Belfort e a/. (1997) Nucleic Acids Res. 25:3379-3388; Ou on et al. (1989) Gene 82 : 115-118 ; Perler et al. ( 1994) Nucleic Acids Res. 22, 1 125- 1 127; Jasin (1996) Trends Genet. 12:224-228; Gimble et al. (1996) J. Mol. Biol. 263: 163- 180; Argast et al. (1998) J Mol. Biol. 280:345-353 and the New England Biolabs catalogue.

In certain embodiments, the nuclease comprises an engineered (non- naturally occurring) homing endonuclease (meganuclease). The recognition sequences of homing endonucieases and meganucleases such as l-Scel, l-Ceul, VI- Pspl, Vl-Sce, l-ScelN, l-Csml, 1-Panl, l-Scell, l-Ppol, l-Scelll, l-Crel, l-Tevl, l-Tevll and I-7evIII are known. See also U.S. Patent No. 5,420,032; U.S. Patent No. 6,833,252; Belfort et al. (1997) Nucleic Acids Res. 25:3379-3388; Dujon ef a/. (1989) Gene 82: 115-118; Perler et al. (1994) Nucleic Acids Res. 22, 1125-1127; Jasin (1996) Trends Genet. 12:224-228; Gimble et al. (1996) J. Mol. Biol. 263: 163- 180; Argast et al. (1998) J. Mol. Biol. 280:345-353 and the New England Biolabs catalogue. In addition, the DNA-binding specificity of homing endonucleases and meganucleases can be engineered to bind non-natural target sites. See, for example, Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66; U.S. Patent Publication No. 20070117128. The DNA-binding domains of the homing endonucleases and meganucleases may be altered in the context of the nuclease as a whole (i.e., such that the nuclease includes the cognate cleavage domain) or may be fused to a heterologous cleavage domain.

In one embodiment, the DNA digesting agent is a site-specific nuclease of the group or selected from the group consisting of omega, zinc finger, TALE, and CRISPR/Cas9.

### D. Markers

In certain embodiments of the invention, cells containing a genomic DNA sequence modification or cells that have been transfected with a composition of the present invention may be identified *in vitro* or *in vivo* by including a marker in the composition. Such markers would confer an identifiable change to the cell permitting easy identification of cells that have been transfected with the composition. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker or an antibiotic resistance gene/marker.

Usually the inclusion of a drug selection marker aids in the cloning and identification of transformants, for example, genes that confer resistance to neomycin, puromycin, hygromycin, DHFR, GPT, zeocin, G418, phleomycin, blasticidin, and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase (*tk*) or chloramphenicol acetyltransferase (CAT) may be utilized. One of skill in the art would also know how to employ immunologic markers, possibly in conjunction with FACS analysis. Further examples of selectable and screenable markers are well known to one of skill in the art. In certain embodiments, the marker is a fluorescent marker, an enzymatic marker, a luminescent marker, a photoactivatable marker, a photoconvertible marker, or a colorimetric marker. Flouorescent markers include, for example, GFP and variants such as YFP, RFP etc., and other fluorescent proteins such as DsRed, mPlum, mCherry, YPet, Emerald, CyPet, T-Sapphire, and Venus. Photoactivatable markers include, for example, KFP, PA-mRFP, and Dronpa. Photoconvertible markers include, for example, mEosFP, KikGR, and PS-CFP2. Luminescent proteins include, for example, Neptune, FP595, and phialidin. Non-limiting examples of screening markers include

The marker used in the invention may be encoded on an RNA or DNA. In a specific embodiment, the marker is encoded on RNA.

In certain aspects, after electroporation cells that have internalized the electroporated compositions are selected for by negative selection. In other aspects, after electroporation cells that have internalized the electroporated constructs are selected for by positive selection. In some aspects selection involves exposing the cells to concentrations of a selection agent that would compromise the viability of a cell that did not express a selection resistance gene or take up a selection resistance gene during electroporation. In some aspects selection involves exposing the cells to a conditionally lethal concentration of the selection agent. In certain aspects the selection agent or compound is an antibiotic. In other aspects the selection agent is G418 (also known as geneticin and G418 sulfate), puromycin, zeocin, hygromycin, phleomycin or blasticidin, either alone or in combination. In certain aspects the concentration of selection agent is in the range of 0.1µg/L to 0.5µg/L, 0.5µg/L to 1µg/L, 1µg/L to 2µg/L, 2µg/L to 5µg/L, 5µg/L to 10µg/L, 10µg/L to 100µg/L, 100µg/L to 500µg/L, 0.1mg/L to 0.5mg/L, 0.5mg/L to 1mg/L, 1mg/L to 2mg/L, 2mg/L to 5mg/L, 5mg/L to 10mg/L, 10mg/L to 100mg/L, 100mg/L to 500mg/L, 0.1g/L to 0.5g/L, 0.5g/L to 1g/L, 1g/L to 2g/L, 2g/L to 5g/L, 5g/L to 10g/L, 10g/L to 100g/L, or 100g/L to 500g/L or any range derivable therein. In certain aspects the concentration of selection agent is (y)g/L, where 'y' can be any value including but not limited to 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, 10, 20, 30, 40, 50, 60, 70, 80, 90, 100 or any range derivable therein. In some embodiments the selection agent is present in the culture media at a conditionally lethal concentration of about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 g/L or any range derivable therein.

In certain embodiments, the nucleic acid segments, regardless of the length of the coding sequence itself, may be combined with other nucleic acid sequences, such as promoters, polyadenylation signals, additional restriction enzyme sites, multiple cloning sites, other coding segments, and the like, such that their overall length may vary considerably.

### E. Vectors

Polypeptides may be encoded by a nucleic acid molecule in the composition. In certain embodiments, the nucleic acid molecule can be in the form of a nucleic acid vector. The term "vector" is used to refer to a carrier nucleic acid molecule into which a heterologous nucleic acid sequence can be inserted for introduction into a cell where it can be replicated and expressed. A nucleic acid sequence can be "heterologous," which means that it is in a context foreign to the cell in which the vector is being introduced or to the nucleic acid in which is incorporated, which includes a sequence homologous to a sequence in the cell or nucleic acid but in a position within the host cell or nucleic acid where it is ordinarily not found. Vectors include DNAs, RNAs, plasmids, cosmids, viruses (bacteriophage, animal viruses, and plant viruses), and artificial chromosomes (e.g., YACs). One of skill in the art would be well equipped to construct a vector through standard recombinant techniques (for example Sambrook et al., 2001; Ausubel et al., 1996). Vectors may be used in a host cell to produce an antibody.

The term "expression vector" refers to a vector containing a nucleic acid sequence coding for at least part of a gene product capable of being transcribed or stably integrate into a host cell's genome and subsequently be transcribed. In some cases, RNA molecules are then translated into a protein, polypeptide, or peptide. Expression vectors can contain a variety of "control sequences," which refer to nucleic acid sequences necessary for the transcription and possibly translation of an operably linked coding sequence in a particular host organism. In addition to control sequences that govern transcription and translation, vectors and expression vectors may contain nucleic acid sequences that serve other functions as well and are described herein. It is contemplated that expression vectors that express a marker may be useful in the invention. In other embodiments, the marker is encoded on an mRNA and not in an expression vector.

A "promoter" is a control sequence. The promoter is typically a region of a nucleic acid sequence at which initiation and rate of transcription are controlled. It may contain genetic elements at which regulatory proteins and molecules may bind such as RNA polymerase and other transcription factors. The phrases "operatively positioned," "operatively linked," "under control," and "under transcriptional control" mean that a promoter is in a correct functional location and/or orientation in relation to a nucleic acid sequence to control transcriptional initiation and expression of that sequence. A promoter may or may not be used in conjunction with an "enhancer," which refers to a cis-acting regulatory sequence involved in the transcriptional activation of a nucleic acid sequence.

The particular promoter that is employed to control the expression of a peptide or protein encoding polynucleotide is not believed to be critical, so long as it is capable of expressing the polynucleotide in a targeted cell, preferably a bacterial cell. Where a human cell is targeted, it is preferable to position the polynucleotide coding region adjacent to and under the control of a promoter that is capable of being expressed in a human cell. Generally speaking, such a promoter might include either a bacterial, human or viral promoter.

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals.

Vectors can include a multiple cloning site (MCS), which is a nucleic acid region that contains multiple restriction enzyme sites, any of which can be used in conjunction with standard recombinant technology to digest the vector. (See Carbonelli *et al.,* 1999, Levenson *et al.,* 1998, and Cocea, 1997.)

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression. (See Chandler *et al.,* 1997.)

The vectors or constructs will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels. In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message.

In expression, particularly eukaryotic expression, one will typically include a polyadenylation signal to effect proper polyadenylation of the transcript.

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

In certain specific embodiments, the composition transfected into the cell by electroporation is non-viral (i.e. does not contain any viral components). It is contemplated that non-viral methods will reduce toxicity and/or improve the safety of the method. It is contemplated that the combination of the use of small DNA oligos and DNA digesting agents provided as RNA provide an advantage of decreased cytotoxicity and increased efficiency of genomic DNA sequence modification.

### F. Nucleic Acid Modifications

In the context of this disclosure, the term "unmodified oligonucleotide" refers generally to an oligomer or polymer of ribonucleic acid (RNA) or deoxyribonucleic acid (DNA). In some embodiments a nucleic acid molecule is an unmodified oligonucleotide. This term includes oligonucleotides composed of naturally occurring nucleobases, sugars and covalent internucleoside linkages. The term "oligonucleotide analog" refers to oligonucleotides that have one or more non-naturally occurring portions which function in a similar manner to oligonucleotides. Such non-naturally occurring oligonucleotides are often selected over naturally occurring forms because of desirable properties such as, for example, enhanced cellular uptake, enhanced affinity for other oligonucleotides or nucleic acid targets and increased stability in the presence of nucleases. The term "oligonucleotide" can be used to refer to unmodified oligonucleotides or oligonucleotide analogs.

Specific examples of nucleic acid molecules include nucleic acid molecules containing modified, i.e., non-naturally occurring internucleoside linkages. Such non-naturally internucleoside linkages are often selected over naturally occurring forms because of desirable properties such as, for example, enhanced cellular uptake, enhanced affinity for other oligonucleotides or nucleic acid targets and increased stability in the presence of nucleases. In a specific embodiment, the modification comprises a methyl group.

Nucleic acid molecules can have one or more modified internucleoside linkages. As defined in this specification, oligonucleotides having modified internucleoside linkages include internucleoside linkages that retain a phosphorus atom and internucleoside linkages that do not have a phosphorus atom. For the purposes of this specification, and as sometimes referenced in the art, modified oligonucleotides that do not have a phosphorus atom in their internucleoside backbone can also be considered to be oligonucleosides.

Modifications to nucleic acid molecules can include modifications wherein one or both terminal nucleotides is modified.

One suitable phosphorus-containing modified internucleoside linkage is the phosphorothioate internucleoside linkage. A number of other modified oligonucleotide backbones (internucleoside linkages) are known in the art and may be useful in the context of this embodiment.

Representative U.S. patents that teach the preparation of phosphorus-containing internucleoside linkages include, but are not limited to, U.S. Pat. Nos. 3,687,808; 4,469,863; 4,476,301; 5,023,243, 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 5,625,050, 5,489,677, and 5,602,240.

Modified oligonucleoside backbones (internucleoside linkages) that do not include a phosphorus atom therein have internucleoside linkages that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having amide backbones; and others, including those having mixed N, O, S and CH2 component parts.

Representative U.S. patents that teach the preparation of the above non-phosphorous-containing oligonucleosides include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439.

Oligomeric compounds can also include oligonucleotide mimetics. The term mimetic as it is applied to oligonucleotides is intended to include oligomeric compounds wherein only the furanose ring or both the furanose ring and the internucleotide linkage are replaced with novel groups, replacement of only the furanose ring with for example a morpholino ring, is also referred to in the art as being a sugar surrogate. The heterocyclic base moiety or a modified heterocyclic base moiety is maintained for hybridization with an appropriate target nucleic acid.

Oligonucleotide mimetics can include oligomeric compounds such as peptide nucleic acids (PNA) and cyclohexenyl nucleic acids (known as CeNA, see Wang et al., J. Am. Chem. Soc., 2000, 122, 8595-8602). Representative U.S. patents that teach the preparation of oligonucleotide mimetics include, but are not limited to, U.S. Pat. Nos. 5,539,082; 5,714,331; and 5,719,262, Another class of oligonucleotide mimetic is referred to as phosphonomonoester nucleic acid and incorporates a phosphorus group in the backbone. This class of olignucleotide mimetic is reported to have useful physical and biological and pharmacological properties in the areas of inhibiting gene expression (antisense oligonucleotides, ribozymes, sense oligonucleotides and triplex-forming oligonucleotides), as probes for the detection of nucleic acids and as auxiliaries for use in molecular biology. Another oligonucleotide mimetic has been reported wherein the furanosyl ring has been replaced by a cyclobutyl moiety.

Nucleic acid molecules can also contain one or more modified or substituted sugar moieties. The base moieties are maintained for hybridization with an appropriate nucleic acid target compound. Sugar modifications can impart nuclease stability, binding affinity or some other beneficial biological property to the oligomeric compounds.

Representative modified sugars include carbocyclic or acyclic sugars, sugars having substituent groups at one or more of their 2', 3' or 4' positions, sugars having substituents in place of one or more hydrogen atoms of the sugar, and sugars having a linkage between any two other atoms in the sugar. A large number of sugar modifications are known in the art, sugars modified at the 2' position and those which have a bridge between any 2 atoms of the sugar (such that the sugar is bicyclic) are particularly useful in this embodiment. Examples of sugar modifications useful in this embodiment include, but are not limited to compounds comprising a sugar substituent group selected from: OH; F; O-, S-, or N-alkyl; or O-alkyl-O-alkyl, wherein the alkyl, alkenyl and alkynyl may be substituted or unsubstituted C1 to C10 alkyl or C2 to C10 alkenyl and alkynyl. Particularly suitable are: 2-methoxyethoxy (also known as 2'-O-methoxyethyl, 2'-MOE, or 2'-OCH2CH2OCH3), 2'-O-methyl (2'-O-CH3), 2'-fluoro (2'-F), or bicyclic sugar modified nucleosides having a bridging group connecting the 4' carbon atom to the 2' carbon atom wherein example bridge groups include - -CH2--O--, --(CH2)2--O-- or --CH2--N(R3)--O wherein R3 is H or C1-C12 alkyl.

One modification that imparts increased nuclease resistance and a very high binding affinity to nucleotides is the 2'-MOE side chain (Baker et al., J. Biol. Chem., 1997, 272, 11944-12000). One of the immediate advantages of the 2'-MOE substitution is the improvement in binding affinity, which is greater than many similar 2' modifications such as O-methyl, O-propyl, and O-aminopropyl. Oligonucleotides having the 2'-MOE substituent also have been shown to be antisense inhibitors of gene expression with promising features for *in vivo* use (Martin, P., Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

2'-Sugar substituent groups may be in the arabino (up) position or ribo (down) position. One 2'-arabino modification is 2'-F. Similar modifications can also be made at other positions on the oligomeric compound, particularly the 3' position of the sugar on the 3' terminal nucleoside or in 2'-5' linked oligonucleotides and the 5' position of 5' terminal nucleotide. Oligomeric compounds may also have sugar mimetics such as cyclobutyl moieties in place of the pentofuranosyl sugar. Representative U.S. patents that teach the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920,

Representative sugar substituents groups are disclosed in U.S. Pat. No. 6,172,209 entitled "Capped 2'-Oxyethoxy Oligonucleotides,"

Representative cyclic sugar substituent groups are disclosed in U.S. Pat. No. 6,271,358 entitled "RNA Targeted 2'-Oligomeric compounds that are Conformationally Preorganized,"

Representative guanidino substituent groups are disclosed in U.S. Pat. No. 6,593,466 entitled "Functionalized Oligomers,"

Representative acetamido substituent groups are disclosed in U.S. Pat. No. 6,147,200

Nucleic acid molecules can also contain one or more nucleobase (often referred to in the art simply as "base") modifications or substitutions which are structurally distinguishable from, yet functionally interchangeable with, naturally occurring or synthetic unmodified nucleobases. Such nucleobase modifications can impart nuclease stability, binding affinity or some other beneficial biological property to the oligomeric compounds. As used herein, "unmodified" or "natural" nucleobases include the purine bases adenine (A) and guanine (G), and the pyrimidine bases thymine (T), cytosine (C) and uracil (U). Modified nucleobases also referred to herein as heterocyclic base moieties include other synthetic and natural nucleobases, many examples of which such as 5-methylcytosine (5-me-C), 5-hydroxymethyl cytosine, 7-deazaguanine and 7-deazaadenine among others.

Heterocyclic base moieties can also include those in which the purine or pyrimidine base is replaced with other heterocycles, for example 7-deaza-adenine, 7-deazaguanosine, 2-aminopyridine and 2-pyridone. Some nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B., ed., CRC Press, 1993. Certain of these nucleobases are particularly useful for increasing the binding affinity of the oligomeric compounds. These include 5-substituted pyrimidines, 6-azapyrimidines and N-2, N-6 and O-6 substituted purines, including 2 aminopropyladenine, 5-propynyluracil and 5-propynylcytosine.

Additional modifications to nucleic acid molecules are disclosed in U.S. Patent Publication 2009/0221685. Also disclosed herein are additional suitable conjugates to the nucleic acid molecules.

### II. Cell culture

### A. Host Cells

As used herein, the terms "cell," "cell line," and "cell culture" may be used interchangeably. All of these terms also include both freshly isolated cells and ex vivo cultured, activated or expanded cells. All of these terms also include their progeny, which is any and all subsequent generations. It is understood that all progeny may not be identical due to deliberate or inadvertent mutations. In the context of expressing a heterologous nucleic acid sequence, "host cell" refers to a prokaryotic or eukaryotic cell, and it includes any transformable organism that is capable of replicating a vector or expressing a heterologous gene encoded by a vector. A host cell can, and has been, used as a recipient for vectors or viruses. A host cell may be "transfected" or "transformed," which refers to a process by which exogenous nucleic acid, such as a recombinant protein-encoding sequence, is transferred or introduced into the host cell. A transformed cell includes the primary subject cell and its progeny.

In certain embodiments electroporation can be carried out on any prokaryotic or eukaryotic cell. In other aspects electroporation involves electroporation of an animal cell. In certain aspects electroporation involves electroporation of a cell line or a hybrid cell type. In some aspects the cell or cells being electroporated are cancer cells, tumor cells or immortalized cells. In some instances tumor, cancer, immortalized cells or cell lines are induced and in other instances tumor, cancer, immortalized cells or cell lines enter their respective state or condition naturally. In certain aspects the cells or cell lines electroporated can be A549, B-cells, B16, BHK-21, C2C12, C6, CaCo-2, CAP/, CAP-T, CHO, CHO2, CHO-DG44, CHO-K1, COS-1, Cos-7, CV-1, Dendritic cells, DLD-1, Embryonic Stem (ES) Cell or derivative, H1299, HEK, 293, 293T, 293FT, Hep G2, Hematopoietic Stem Cells, HOS, Huh-7, Induced Pluripotent Stem (iPS) Cell or derivative, Jurkat, K562, L5278Y, LNCaP, MCF7, MDA-MB-231, MDCK, Mesenchymal Cells, Min-6, Monocytic cell, Neuro2a, NIH 3T3, NIH3T3L1, K562, NK-cells, NS0, Panc-1, PC12, PC-3, Peripheral blood cells, Plasma cells, Primary Fibroblasts, RBL, Renca, RLE, SF21, SF9, SH-SY5Y, SK-MES-1, SK-N-SH, SL3, SW403, Stimulus-triggered Acquisition of Pluripotency (STAP) cell or derivate SW403, T-cells, THP-1, Tumor cells, U2OS, U937, peripheral blood lymphocytes, expanded T cells, hematopoietic stem cells, or Vero cells. In some embodiments, the cells are peripheral blood lymphocytes, expanded T cells, stem cells, hematopoietic stem cells, or primary cells. In some embodiments, the cells are hematopoietic stem cells. In some embodiment, the cells are peripheral blood lymphocytes.

In some embodiments, the cells are cells isolated from a patient. In some embodiments, the cells are freshly isolated. In some embodiments, the cells are transfected at a time period of less than or exactly 20, 19, 18, 17, 16, 15, 14, 13, 12, 11, 10, 9, 8, 7, 6, 5, 4, 3, 2, 1 days or less than or exactly 24, 22, 20, 18, 16, 14, 12, 10, 8, 6, 4, 2, 1 hours or any derivable range therein. In some embodiments, the isolated cells have never been frozen. In some embodiments, the isolated cells have never been passaged in vitro. In some embodiments, the isolated cells have been passaged for less than or exactly 10, 9, 8, 7, 6, 5, 4, 3, 2, 1, time, or any derivable range there. The term "passaged" is intended to refer to the process of splitting cells in order to produce large number of cells from pre-existing ones. Passaging involves splitting the cells and transferring a small number into each new vessel. For adherent cultures, cells first need to be detached, commonly done with a mixture of trypsin-EDTA. A small number of detached cells can then be used to seed a new culture, while the rest is discarded. Also, the amount of cultured cells can easily be enlarged by distributing all cells to fresh flasks.

In certain embodiments, the cell is one that is known in the art to be difficult to transfect. Such cells are known in the art and include, for example, primary cells, insect cells, SF9 cells, Jurkat cells, CHO cells, stem cells, slowly dividing cells, and non-dividing cells. In some embodiments, the cell is a germ cell such as an egg cell or sperm cell. In some embodiments, the cell is a fertilized embryo.

In some embodiments, cells may subjected to limiting dilution methods to enable the expansion of clonal populations of cells. The methods of limiting dilution cloning are well known to those of skill in the art. Such methods have been described, for example for hybridomas but can be applied to any cell. Such methods are described in (Cloning hybridoma cells by limiting dilution, Journal of tissue culture methods, 1985, Volume 9, Issue 3, pp 175-177, by Joan C. Rener, Bruce L. Brown, and Roland M. Nardone)

In some embodiments cells are cultured before electroporation or after electroporation. In other embodiments, cells are cultured during the selection phase after electroporation. In yet other embodiments, cells are cultured during the maintenance and clonal selection and initial expansion phase. In still other embodiments, cells are cultured during the screening phase. In other embodiments, cells are cultured during the large scale production phase. Methods of culturing suspension and adherent cells are well-known to those skilled in the art. In some embodiments, cells are cultured in suspension, using commercially available cell-culture vessels and cell culture media. Examples of commercially available culturing vessels that may be used in some embodiments including ADME/TOX Plates, Cell Chamber Slides and Coverslips, Cell Counting Equipment, Cell Culture Surfaces, Corning HYPERFlask Cell Culture Vessels, Coated Cultureware, Nalgene Cryoware, Culture Chamber, Culture Dishes, Glass Culture Flasks, Plastic Culture Flasks, 3D Culture Formats, Culture Multiwell Plates, Culture Plate Inserts, Glass Culture Tubes, Plastic Culture Tubes, Stackable Cell Culture Vessels, Hypoxic Culture Chamber, Petri dish and flask carriers, Quickfit culture vessels, Scale-Up Cell Culture using Roller Bottles, Spinner Flasks, 3D Cell Culture, or cell culture bags.

In other embodiments, media may be formulated using components well-known to those skilled in the art. Formulations and methods of culturing cells are described in detail in the following references: Short Protocols in Cell Biology J. Bonifacino, et al., ed., John Wiley & Sons, 2003, 826 pp; Live Cell Imaging: A Laboratory Manual D. Spector & R. Goldman, ed., Cold Spring Harbor Laboratory Press, 2004, 450 pp.; Stem Cells Handbook S. Sell, ed., Humana Press, 2003, 528 pp.; Animal Cell Culture: Essential Methods, John M. Davis, John Wiley & Sons, Mar 16, 2011; Basic Cell Culture Protocols, Cheryl D. Helgason, Cindy Miller, Humana Press, 2005; Human Cell Culture Protocols, Series: Methods in Molecular Biology, Vol. 806, Mitry, Ragai R.; Hughes, Robin D. (Eds.), 3rd ed. 2012, XIV, 435 p. 89, Humana Press; Cancer Cell Culture: Method and Protocols, Cheryl D. Helgason, Cindy Miller, Humana Press, 2005; Human Cell Culture Protocols, Series: Methods in Molecular Biology, Vol. 806, Mitry, Ragai R.; Hughes, Robin D. (Eds.), 3rd ed. 2012, XIV, 435 p. 89, Humana Press; Cancer Cell Culture: Method and Protocols, Simon P. Langdon, Springer, 2004; Molecular Cell Biology. 4th edition., Lodish H, Berk A, Zipursky SL, et al., New York: W. H. Freeman; 2000., Section 6.2Growth of Animal Cells in Culture,

In some embodiments, during the screening and expansion phase and/or during the large scale production phase (also referred to as fed-batch & comparison), expanded electroporated cells that result from selection or screening may comprise modified genomic DNA sequence.

### III. Therapeutic and drug discovery applications

In certain embodiments, the cells and cell lines produced by methods described herein are ones that, upon modification of the genomic DNA, provide a therapeutic effect. Primary cells may be isolated, modified by methods described herein, and used ex vivo for reintroduction into the subject to be treated. Suitable primary cells include peripheral blood mononuclear cells (PBMC), peripheral blood lmyphocytes (PBLs) and other blood cell subsets such as, but not limited to, CD4+ T cells or CD8+ T cells. Other suitable primary cells include progenitor cells such as myeloid or lymphoid progenitor cells. Suitable cells also include stem cells such as, by way of example, embryonic stem cells, induced pluripotent stem cells, hematopoietic stem cells, neuronal stem cells, mesenchymal stem cells, muscle stem cells and skin stem cells. For example, iPSCs can be derived ex vivo from a patient afflicted with a known genetic mutation associated, and this mutation can be modified to a wild-type allele using methods described herein. The modified iPSC can then be differentiated into dopaminergic neurons and reimplanted into the patient. In another ex vivo therapeutic application, hematopoietic stem cells can be isolated from a patient afflicted with a known genetic mutation, which can then be modified to correct the genetic mutation. The HSCs can then be administered back to the patient for a therapeutic effect or can be differentiated in culture into a more mature hematopoietic cell prior to administration to the patient.

In some embodiments, the modified genomic DNA sequence comprises a disease-associated gene. Disease-associated genes are known in the art. It is contemplated that a disease associated gene is one that is disclosed on the world wide web at genecards.org/cgi-bin/listdiseasecards.pl?type=full&no_limit=1.

In some embodiments, the method comprises modifying genomic DNA in hematopoietic stem cells (a.k.a. hemocytoblasts) or in myeloid progenitor cells.

In some embodiments, the method comprises modifying the *HBB* gene genomic DNA sequence in hematopoietic stem cells (a.k.a. hemocytoblasts) or in myeloid progenitor cells. In certain embodiments, the sequence is modified to correct a disease-associated mutation in the genomic sequence. For example, the genomic sequence of a subject with sickle-cell anemia may be modified to correct the E6V mutation. Therefore, methods described herein may be used to correct the genomic sequence of cells from a subject harboring a genomic mutation that produces a β-globin protein with a valine at the sixth position instead of a glutamic acid. Accordingly, in one embodiment, the sequence modification is the correction of the genomic DNA that modifies the sixth codon of the *HBB* gene to a glutamic acid codon.

The protein coding sequence for the *HBB* gene is exemplified in SEQ ID NO: 19:
MVHLTPEEKSAVTALWGKVNVDEVGGEALGRLLVVYPWTQRFFESFGDLSTPDAV MGNPKVKAHGKKVLGAFSDGLAHLDNLKGTFATLSELHCDKLHVDPENFRLLGNV LVCVLAHHFGKEFTPPVQAAYQKVVAGVANALAHKYH. The underlined glutamic acid represents the amino acid at the sixth position of the protein. At the DNA level, the genomic DNA comprises a GAG to GTG mutation, which results in the E6V mutant protein.

In some embodiments, the method comprises modifying the gp91phox gene in cells. In some embodiments, the cells are autologous cells from the patient. In some embodiments, the cells are hematopoietic stem cells. In some embodiments, the method is for treating chronic granulomatous disease (CGD).

The protein coding sequence for the gp91phox gene is exemplified by SEQ ID NO:20: mgnwavnegl sifvilvwlg lnvflfvwyy rvydippkff ytrkllgsal alarapaacl nfncmlillp vcrnllsfir gssaccstrv rrqldrnltf hkmvawmial hsaihtiahl fnvewcvnar vnnsdpysva lselgdrqne sylnfarkri knpegglyla vtllagitgv vitlclilii tsstktirrs yfevfwythh lfviffigla ihgaerivrg qtaeslavhn itvceqkise wgkikecpip qfagnppmtw kwivgpmfly lcerlvrfwr sqqkwitkv vthpfktiel qmkkkgfkme vgqyifvkcp kvsklewhpf tltsapeedf fsihirivgd wteglfnacg cdkqefqdaw klpkiavdgp fgtasedvfs yevvmlvgag igvtpfasil ksvwykycnn atnlklkkiy fywlcrdtha fewfadllql lesqmqernn agflsyniyl tgwdesqanh favhhdeekd vitglkqktl ygrpnwdnef ktiasqhpnt rigvflcgpe alaetlskqs isnsesgprg vhfifnkenf (SEQ ID NO: 20).

In some embodiments, the method corrects the nucleotide sequence of the gp91phox gene at Exon 7, position 676C to T. Correcting nucleotide "T" at amino acid site 226 of gp91phox to be "C" restores the site to be the right Arg from the stop codon. Accordingly, in one embodiment, the sequence modification is the correction of the genomic DNA that modifies the 226th codon of the gp91phox gene to an Arg codon.

In certain aspects, the methods described herein relate to an improved method for ex vivo therapy. A population of cells may be isolated from a subject, and the genomic DNA of the cells may be modified in a manner that corrects a defect. The population of cells may then be transplanted into a subject for therapeutic use. In certain instances, the population of cells isolated may comprise a subset of cells sensitive to certain in vitro manipulations such as traditional transfection and/or electroporation methods, for example, or the subset of cells may be resistant to traditional transfection and/or electroporation methods or genomic DNA manipulation. It is contemplated that modifying the genomic DNA with methods described herein will result in a greater efficiency of the sequence modification in such populations.

The efficiency of the sequence modification may also be referred to herein as the editing rate. This can be calculated by number of cells edited divided by the total number of cells. In the examples provided herein, the editing rate was calculated as (density of digested bands)/[(density of digested bands + density of parental band).

One aspect of the disclosure relates to a method for site-specific sequence modification or amendment of a target genomic DNA region in cells isolated from a subject comprising: isolating cells from a subject; transfecting the cells by electroporation with a composition comprising (a) a DNA oligo and (b) a DNA digesting agent; wherein the donor DNA comprises: (i) a homologous region comprising nucleic acid sequence homologous to the target genomic DNA region; and (ii) a sequence modification region; and wherein the genomic DNA sequence is modified specifically at the target genomic DNA region. In one embodiment, the isolated cells comprise two or more different cell types.

When used in this context, the term "different cell types" may mean cells which originate from different cell lineages or cells which originate from the same lineage, but are at a different stage of pluripotency or differentiation. In one embodiment, the two or more different cell types comprise two or more cell types at different stages of pluripotency or differentiation. In a further embodiment, the cells are from the same lineage, but are at a different stage of pluripotency or differentiation.

It is contemplated that the methods described herein will not be negatively selective to certain populations of cells. Accordingly, in certain embodiments, the efficiency of the sequence modification between the two or more different cell types is less than 1% different. In further embodiments, the efficiency of the sequence modification between the two or more cell types is less than 2, 1.5, 1, 0.5, 0.1, 0.05, or 0.01 % different. In other embodiments, the cell viability is less than 5% different between the two or more cell types. In further embodiments, the cell viability is less than 10, 7, 3, 2, 1, 0.5, or 0.1 % different between the two cell populations.

In specific embodiments, the isolated cells are cells isolated from the bone marrow of the subject. In a further embodiment, the isolated cells comprise stem cells. The stem cells may be any stem cell isolatable from the body. Non-limiting examples include hematopoietic stem cells, mesenchymal stem cells, and neural stem cells. In a specific embodiment, the cell is a hematopoietic stem cells. In a further embodiment, the isolated cells comprise the cell surface marker CD34+.

Additionally, cells and cell lines produced by the methods used herein may be useful for drug development and/or reverse genetic studies. Such cells and animals may reveal phenotypes associated with a particular mutation or with its sequence modification, and may be used to screen drugs that will interact either specifically with the mutation(s) or mutant proteins in question, or that are useful for treatment of the disease in an afflicted animal. These cell lines can also provide tools to investigate the effects of specific mutations since a cell line and its corresponding "modified" cell line represent "genetically identical" controls and thus provides a powerful tool for repair of disease-specific mutations, drug screening and discovery, and disease mechanism research. It is further contemplated that this technology can provide a scientifically superior alternative to current gene-knockdown techniques such as RNAi and shRNAs, for example. In one example, a the DNA sequence modification is a stop codon that is introduced into a gene of interest to study a developmental or disease mechanism or for a therapeutic application.

### IV. Electroporation

Certain embodiments involve the use of electroporation to facilitate the entry of one or more nucleic acid molecules into host cells.

As used herein, "electroporation" or "electroloading" refers to application of an electrical current or electrical field to a cell to facilitate entry of a nucleic acid molecule into the cell. One of skill in the art would understand that any method and technique of electroporation is contemplated by the present invention.

In certain embodiments of the invention, electroloading may be carried out as described in U.S. Patent number 5,612,207 U.S. Patent number 5,720,921 U.S. Patent number 6,074,605 U.S. Patent number 6,090,617; U.S. patent number 6,485,961; U.S. patent number 7,029,916 U.S. patent number 7,141,425, U.S. patent number 7,186,559 U.S. patent number 7,771,984 and U.S. publication number 2011/0065171.

Other methods and devices for electroloading that may be used in the context of the present invention are also described in, for example, published PCT Application Nos. WO 03/018751 and WO 2004/031353; US Patent Application Nos. 10/781,440, 10/080,272, and 10/675,592; and US Patent Nos. 6,773,669, 6,090,617, 6,617,154

In certain embodiments of the invention, electroporation may be carried out as described in U.S. Patent application serial no. 10/225,446, filed August 21, 2002

In further embodiments of the invention, flow electroporation is performed using MaxCyte STX^{®}, MaxCyte VLX^{®}, or MaxCyte GT^{®} flow electroporation instrumentation. In specific embodiments, static or flow electroporation is used with parameters described throughout the disclosure.

The claimed methods of transfecting cells by electroporation, preferably flow electroporation, is capable of achieving transfection efficiencies of greater than 40%, greater than 50% and greater than 60%, 70%, 80% or 90% (or any range derivable therein). Transfection efficiency can be measured either by the percentage of the cells that express the product of the gene or the secretion level of the product express by the gene. The cells maintain a high viability during and after the electroporation process. Viability is routinely more than 50% or greater. Viability or electroporated cells can be at most or at least about 5%, 10%, 15%, 20%, 25%, 30%, 35%, 40%, 45%, 50%, 55%, 60%, 65%, 70%, 75%, 80%, 85%, 90% or 95% (or any range derivable therein). of the viability of the starting, unelectroporated population or an electroporated population transfected with a control construct.

In some embodiments the current methods use a flow electroporation apparatus for electrical stimulation of suspensions of particles, comprising a flow electroporation cell assembly having one or more inlet flow portals, one or more outlet flow portals, and one or more flow channels, the flow channels being comprised of two or more walls, with the flow channels further being configured to receive and transiently contain a continuous flow of particles in suspension from the inlet flow portals; and paired electrodes disposed in relation to the flow channels such that each electrode forms at least one wall of the flow channels, the electrodes further comprising placing the electrodes in electrical communication with a source of electrical energy, whereby suspensions of particles flowing through the channels may be subjected to an electrical field formed between the electrodes.

In some embodiments the current methods use flow electroporation to overcome the limitation of sample size. With this method, a cell suspension is passed across parallel bar electrodes that are contained in a flow cell that is preferably disposable.

In further embodiments, the flow or static electroporation methods described herein are employed to overcome thermal degradation of the sample. It is to be understood that different configurations of cells can be used in the current methods. During electroporation, the cells are subjected to electrical pulses with predetermined characteristics. For example, the specific settings for preparation of sample cells are: voltage, 750V; pulse width, 650 µsec; time between pulses, 100 µsec; 2 biphasic pulses in a burst; time between bursts, 12 sec; flow rate, 0.05 mL/sec. The molecule or molecules of interest can then diffuse into the cell following concentration and/or electrical gradients. The present invention is optionally capable of subjecting the cells to a range of electric field strengths.

Another advantage of the current flow electroporation methods is the speed at which a large population of cells can be transfected. For example, a population of lymphocytes can be transfected by electroporation by electroporating the sample in less than 5 hours, preferably less than 4 hours and most preferable in less than 3 hours and most preferably in less than 2 hours. The time of electroporation is the time that the sample is processed by the flow electroporation process. In certain embodiments, 1E10 cells are transfected in 30 minutes or less using flow electroporation. In further embodiments, 2E11 cells may be transfected in 30 minutes, or 60 minutes or less using flow electroporation.

For flow electroporation, the process is initiated by attaching the flow cell with solutions and cell suspensions in the containers with the necessary fluids and samples. Priming solution (saline) and cell suspension are introduced by providing the required commands to the electroporation system, which controls operation of the pump and pinch valves. As the cells transit the flow path between electrodes, electric pulses of the chosen voltage, duration, and frequency are applied. Product and waste fluids are collected in the designated containers.

The user inputs the desired voltage and other parameters into the flow electroporation system of the present invention. As noted above, a range of settings is optionally available. The computer communicates to the electronics in the tower to charge the capacitor bank to the desired voltage. Appropriate switches then manipulate the voltage before it is delivered to the flow path to create the electric field (the switches provide alternating pulses or bursts to minimize electrode wear brought on by prolonged exposure to the electric field). The voltage is delivered according to the duration and frequency parameters set into the flow electroporation system of the present invention by the operator. The flow electroporation system of the present invention is now described in detail.

The flow electroporation process can be initiated by, for example, placing an electroporation chamber in fluid communication with solutions and cell suspensions in containers (e.g., via tubing), which may be carried out in an aseptic or sterile environment. A cell suspension and/or other reagents may be introduced to the electroporation chamber using one or more pumps, vacuums, valves, other mechanical devices that change the air pressure or volume inside the electroporation chamber and combinations thereof, which can cause the cell suspension and/or other reagents to flow into the electroporation chamber at a desired time and at the desired rate. If a portion of the cell suspension and/or other reagents is positioned in the electroporation chamber, electric pulses of a desired voltage, duration, and/or interval are applied the cell suspension and/or other reagents. After electroporation, the processed cell suspension and/or other reagents can be removed from the electroporation chamber using one or more pumps, vacuums, valves, other electrical, mechanical, pneumatic, or microfluidic devices that change the displacement, pressure or volume inside the electroporation chamber, and combinations thereof. In certain embodiments, gravity or manual transfer may be used to move sample or processed sample into or out of an electroporation chamber. If desired, a new cell suspension and/or other reagents can be introduced into the electroporation chamber. An electroporated sample can be collected separately from a sample that has not yet been electroporated. The preceding series of events can be coordinated temporally by a computer coupled to, for example, electronic circuitry (e.g., that provides the electrical pulse), pumps, vacuums, valves, combinations thereof, and other components that effect and control the flow of a sample into and out of the electroporation chamber. As an example, the electroporation process can be implemented by a computer, including by an operator through a graphic user interface on a monitor and/or a keyboard. Examples of suitable valves include pinch valves, butterfly valves, and/or ball valves. Examples of suitable pumps include centrifugal or positive displacement pumps.

As an example, a flow electroporation device can comprise at least two electrodes separated by a spacer, where the spacer and the at least two electrodes define a chamber. In some embodiments, the electroporation chamber can further comprise a least three ports traversing the spacer, where a first port is for sample flow into the chamber, a second port is for processed sample flow out of the chamber, and a third port is for non-sample fluid flow into or out of the chamber. In some embodiments, the non-sample fluid flows out of the chamber when a sample flows into the chamber, and the non-sample fluid flows into the chamber when processed sample flows out of the chamber. As another example, a flow electroporation device can comprise an electroporation chamber having a top and bottom portion comprising at least two parallel electrodes, the chamber being formed between the two electrodes and having two chamber ports in the bottom portion of the electroporation chamber and two chamber ports in the top portion of the electroporation chamber. Such a device can further comprise at least one sample container in fluid communication with the electroporation chamber through a first chamber port in the bottom portion of the chamber, and the electroporation chamber can be in fluid communication with the sample container through a second chamber port in the top portion of the chamber, forming a first fluid path. Further, at least one product container can be in fluid communication with the electroporation chamber through third chamber port in the bottom portion of the chamber, and the electroporation chamber can be in fluid communication with the product container through a fourth chamber port in the top portion of the chamber, forming a second fluid path. In some embodiments, a single port electroporation chamber may be used. In other embodiments, various other suitable combinations of electrodes, spacers, ports, and containers can be used. The electroporation chamber can comprise an internal volume of about 1-10 mL; however, in other embodiments, the electroporation chamber can comprise a lesser internal volume (e.g., 0.75 mL, 0.5 mL, 0.25 mL, or less) or a greater internal volume (e.g., 15 mL, 20 mL, 25 mL, or greater). In some embodiments, the electroporation chamber and associated components can be disposable (e.g., Medical Grade Class VI materials), such as PVC bags, PVC tubing, connectors, silicone pump tubing, and the like.

Any number of containers (e.g., 1, 2, 3, 4, 5, 6, or more) can be in fluid communication with the electroporation chamber. The containers may be a collapsible, expandable, or fixed volume containers. For example, a first container (e.g., a sample source or sample container) can comprise a cell suspension and may or may not include a substance that will pass into cells in the cell suspension during electroporation. If the substance is not included, a second container comprising this substance can be included such that the substance can be mixed inline before entry into the electroporation chamber or in the electroporation chamber. In an additional configuration, another container may be attached, which can hold fluid that will be discarded. One or more additional containers can be used as the processed sample or product container. The processed sample or product container will hold cells or other products produced from the electroporation process. Further, one or more additional containers can comprise various non-sample fluids or gases that can be used to separate the sample into discrete volumes or unit volumes. The non-sample fluid or gas container can be in fluid communication with the electroporation chamber through a third and/or fourth port. The non-sample fluid or gas container may be incorporated into the processed sample container or the sample container (e.g., the non-sample fluid container can comprise a portion of the processed sample container or the sample container); and thus, the non-sample fluid or gas can be transferred from the processed sample container to another container (which may include the sample container) during the processing of the sample. The non-sample fluid or gas container may be incorporated into the chamber, as long as the compression of the non-sample fluid or gas does not affect electroporation. Further aspects of the invention may include other containers that are coupled to the sample container and may supply reagents or other samples to the chamber.

In further embodiments, the electroporation device is static electroporation and does not involve a flow of cells, but instead involves a suspension of cells in a single chamber. When such device is employed, the parameters described for flow electroporation may be used to limit thermal degradation, improve cell viability, improve efficiency of sequence modification incorporation, improve transfection efficiency and the like. Such parameters include, for example, the flow electroporation parameters described throughout the application and thermal resistance of the chamber, spacing of electrodes, ratio of combined electrode surface in contact with buffer to the distance between the electrodes, and electric field.

It is specifically contemplated that embodiments described herein may be excluded. It is further contemplated that, when a range is described, certain ranges may be excluded.

In certain aspects the density of cells during electroporation is a controlled variable. The cell density of cells during electroporation may vary or be varied according to, but not limited to, cell type, desired electroporation efficiency or desired viability of resultant electroporated cells. In certain aspects the cell density is constant throughout electroporation. In other aspects cell density is varied during the electroporation process. In certain aspects cell density before electroporation may be in the range of 1×10⁴ cells/mL to (y)×10⁴, where y can be 2, 3, 4, 5, 6, 7, 8, 9, or 10. In other aspects the cell density before electroporation may be in the range of 1×10⁵ cells/mL to (y)×10⁵, where y is 2, 3, 4, 5, 6, 7, 8, 9, or 10 (or any range derivable therein). In yet other aspects the cell density before electroporation may be in the range of 1×10e6 cells/mL to (y)×10⁶, where y can be 2, 3, 4, 5, 6, 7, 8, 9, or 10. In certain aspects cell density before electroporation may be in the range of 1×10⁷ cells/mL to (y)×10⁷, where y can be 2, 3, 4, 5, 6, 7, 8, 9, or 10 or any range derivable therein. In yet other aspects the cell density before electroporation may be in the range of 1×10⁷ cells/mL to 1×10⁸ cells/mL, 1×10⁸ cells/mL to 1×10⁹ cells/mL, 1x10" cells/mL to 1×10¹⁰ cells/mL, 1×10¹⁰ cells/mL to 1×10¹¹ cells/mL, or 1×10¹¹ cells/mL to 1×10¹² cells/mL. In certain aspects the cell density before electroporation may be (y)×10⁶, where y can be any of 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, 1, 1, 2, 3, 4, 5, 6, 7, 8, 9, 10, 10, 20, 30, 40, 50, 60, 70, 80, 90 or 100 or any range derivable therein. In certain aspects the cell density before electroporation may be (y)×10¹⁰, where y can be any of 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, 1, 1, 2, 3, 4, 5, 6, 7, 8, 9, 10, 10, 20, 30, 40, 50, 60, 70, 80, 90, 100, 200, 300, 400, 500, 600, 700, 800, 900, or 1000 (or any range derivable therein).

In certain aspects the density of cells during electroporation is a controlled variable. The cell density of cells during electroporation may vary or be varied according to, but not limited to, cell type, desired electroporation efficiency or desired viability of resultant electroporated cells. In certain aspects the cell density is constant throughout electroporation. In other aspects cell density is varied during the electroporation process. In certain aspects cell density during electroporation may be in the range of 1×10⁴ cells/mL to (y)×10⁴, where y can be 2, 3, 4, 5, 6, 7, 8, 9, or 10 (or any range derivable therein). In other aspects the cell density during electroporation may be in the range of 1×10⁵ cells/mL to (y)×10⁵, where y is 2, 3, 4, 5, 6, 7, 8, 9, or 10 (or any range derivable therein). In yet other aspects the cell density during electroporation may be in the range of 1×10⁶ cells/mL to (y)×10⁶, where y can be 2, 3, 4, 5, 6, 7, 8, 9, or 10 (or any range derivable therein). In certain aspects cell density during electroporation may be in the range of 1×10⁷ cells/mL to (y)×10⁷, where y can be 2, 3, 4, 5, 6, 7, 8, 9, or 10 (or any range derivable therein). In yet other aspects the cell density during electroporation may be in the range of 1×10⁷ cells/mL to 1×10⁸ cells/mL, 1×10⁸ cells/mL to 1×10⁹ cells/mL, 1x10" cells/mL to 1×10¹⁰ cells/mL, 1×10¹⁰ cells/mL to 1×10¹¹ cells/mL, or 1×10¹¹ cells/mL to 1×10¹² cells/mL. In certain aspects the cell density during electroporation may be (y)×10⁶, where y can be any of 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, 1, 1, 2, 3, 4, 5, 6, 7, 8, 9, 10, 10, 20, 30, 40, 50, 60, 70, 80, 90 or 100 (or any range derivable therein). In certain aspects the cell density during electroporation may be (y)×10¹⁰, where y can be any of 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, 1, 1, 2, 3, 4, 5, 6, 7, 8, 9, 10, 10, 20, 30, 40, 50, 60, 70, 80, 90, 100, 200, 300, 400, 500, 600, 700, 800, 900, or 1000 (or any range derivable therein).

In certain aspects cell density after electroporation may be in the range of 1×10⁴ cells/mL to (y)×10⁴, where y can be 2, 3, 4, 5, 6, 7, 8, 9, or 10 (or any range derivable therein). In other aspects the cell density after electroporation may be in the range of 1×10⁵ cells/mL to (y)×10⁵, where y is 2, 3, 4, 5, 6, 7, 8, 9, or 10 (or any range derivable therein). In yet other aspects the cell density after electroporation may be in the range of 1×10⁶ cells/mL to (y)×10⁶, where y can be 2, 3, 4, 5, 6, 7, 8, 9, or 10 (or any range derivable therein). In certain aspects cell density after electroporation may be in the range of 1×10⁷ cells/mL to (y)×10⁷, where y can be 2, 3, 4, 5, 6, 7, 8, 9, or 10 (or any range derivable therein). In yet other aspects the cell density after electroporation may be in the range of 1×10⁷ cells/mL to 1×10⁸ cells/mL, 1×10⁸ cells/mL to 1×10⁹ cells/mL, 1x10" cells/mL to 1×10¹⁰ cells/mL, 1×10¹⁰ cells/mL to 1×10¹¹ cells/mL, or 1×10¹¹ cells/mL to 1×10¹² cells/mL (or any range derivable therein). In certain aspects the cell density after electroporation may be (y)x10e6, where y can be any of 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, 1, 1, 2, 3, 4, 5, 6, 7, 8, 9, 10, 10, 20, 30, 40, 50, 60, 70, 80, 90 or 100 (or any range derivable therein). In certain aspects the cell density after electroporation may be (y)×10¹⁰, where y can be any of 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, 1, 1, 2, 3, 4, 5, 6, 7, 8, 9, 10, 10, 20, 30, 40, 50, 60, 70, 80, 90, 100, 200, 300, 400, 500, 600, 700, 800, 900, or 1000 (or any range derivable therein).

In certain embodiments electroporation can be carried out on any prokaryotic or eukaryotic cell. In some aspects electroporation involves electroporation of a human cell. In other aspects electroporation involves electroporation of an animal cell. In certain aspects electroporation involves electroporation of a cell line or a hybrid cell type. In some aspects the cell or cells being electroporated are cancer cells, tumor cells or immortalized cells. In some instances tumor, cancer, immortalized cells or cell lines are induced and in other instances tumor, cancer, immortalized cells or cell lines enter their respective state or condition naturally. In certain aspects the cells or cell lines electroporated can be A549, B-cells, B16, BHK-21, C2C12, C6, CaCo-2, CAP/, CAP-T, CHO, CHO2, CHO-DG44, CHO-K1, CHO-DUXB11 COS-1, Cos-7, CV-1, Dendritic cells, DLD-1, Embryonic Stem (ES) Cell or derivative, H1299, HEK, 293, 293T, 293FT, Hep G2, Hematopoietic Stem Cells, HOS, Huh-7, Induced Pluripotent Stem (iPS) Cell or derivative, Jurkat, K562, L5278Y, LNCaP, MCF7, MDA-MB-231, MDCK, Mesenchymal Cells, Min-6, Monocytic cell, Neuro2a, NIH 3T3, NIH3T3L1, NK-cells, NS0, Panc-1, PC12, PC-3, Peripheral blood cells, Plasma cells, Primary Fibroblasts, RBL, Renca, RLE, SF21, SF9, SH-SY5Y, SK-MES-1, SK-N-SH, SL3, SW403, Stimulus-triggered Acquisition of Pluripotency (STAP) cell or derivate SW403, T-cells, THP-1, Tumor cells, U2OS, U937, or Vero cells.

In certain embodiments, the cell is one that is known in the art to be difficult to transfect. Such cells are known in the art and include, for example, primary cells, insect cells, SF9 cells, Jurkat cells, CHO cells, stem cells, slowly dividing cells, and non-dividing cells.

In some instances certain number of cells can be electroporated in a certain amount of time. Given the flexibility, consistency and reproducibility of the described platform up to or more than about (y)×10⁴, (y)×10⁵, (y)×10⁶, (y)×10⁷, (y)×10⁸, (y)×10⁹, (y)×10¹⁰, (y)×10¹¹, (y)×10¹² , (y)×10¹³ , (y)×10¹⁴ , or (y)×10¹⁵ cells (or any range derivable therein) can be electroporated, where y can be any of 1, 2, 3, 4, 5, 6, 7, 8, or 9 (or any range derivable therein). in less than 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, 1, 1, 2, 3, 4, 5, 6, 7, 8, 9, 10, 10, 20, 30, 40, 50, 60, 70, 80, 90 or 100 seconds (or any range derivable therein). In other instances, up to or more than about (y)×10⁴, (y)×10⁵, (y)×10⁶, (y)×10⁷, (y)×10⁸, (y)×10⁹, (y)×10¹⁰, (y)×10¹¹, (y)×10¹² , (y)×10¹³ , (y)×10¹⁴ , or (y)×10¹⁵ cells (or any range derivable therein) can be electroporated, where y can be any of 1, 2, 3, 4, 5, 6, 7, 8, or 9 (or any range derivable therein), in less than 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, 1, 1, 2, 3, 4, 5, 6, 7, 8, 9, 10, 10, 20, 30, 40, 50, 60, 70, 80, 90, 100. 110, or 120 minutes (or any range derivable therein). In yet other aspects, up to or more than about (y)×10⁴, (y)×10⁵, (y)×10⁶, (y)×10⁷, (y)×10⁸, (y)×10⁹, (y)×10¹⁰, (y)×10¹¹, (y)×10¹² , (y)×10¹³ , (y)×10¹⁴ , or (y)×10¹⁵ cells (or any range derivable therein) can be electroporated, where y can be any of 1, 2, 3, 4, 5, 6, 7, 8, or 9 (or any range derivable therein). in less than 1, 2, 3, 4, 5, 6, 7, 8, 9, 10, 11, 12, 13, 14, 15, 16, 17, 18, 19, 20, 21, 22, 23, or 24 hours (or any range derivable therein).

The expression '(y)×10^{e}' is understood to mean, a variable 'y' that can take on any numerical value, multiplied by 10 that is raised to an exponent value, e. For example, (y)×10⁴, where y is 2, is understood to mean 2×10⁴, which is equivalent to 2x10,000, equal to 20,000. (y)x10e4 can also be written as (y)*10e4 or (y) × 10⁴ or (y)^{∗}10⁴.

Volumes of cells or media may vary depending on the amount of cells to be electroporated, the number of cells to be screened, the type of cells to be screened, the type of protein to be produced, amount of protein desired, cell viability, and certain cell characteristics related to desirable cell concentrations. Examples of volumes that can be used in methods and compositions include, but are not limited to, 0.01, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 ml or L (or any range derivable therein), and any range derivable therein. Containers that may hold such volumes are contemplated for use in embodiments described herein. Such containers include, but are not limited to, cell culture dishes, petri dishes, flasks, biobags, biocontainers, bioreactors, or vats. Containers for large scale volumes are particularly contemplated, such as those capable of holding greater than 10L or more. In certain embodiments, volumes of 100 L or more are used.

It is specifically contemplated that electroporation of cells by methods described herein provide benefits of increased efficiency and/or reduced toxicity. Such measurements may be made by measuring the amount of cells that incorporated the genomic DNA sequence modification, measuring the amount of cells that express the marker, and/or measuring the viability of the cells after electroporation.

In some embodiments, the efficiency of the sequence modification is greater than about 2, 3, 4, 5, 6, 7, 8, 9, 10, 15, 20, 25, 30, 35, 40, 45, 50, or 80%. The efficiency of the sequence modification can be measured by determining the number of cells with the sequence modification and dividing by the total number of cells. Incorporation of the genome DNA sequence modification can be determined by methods known in the art such as direct genomic DNA sequencing, differential restriction digestion (if the sequence modification adds, removes, or changes a restriction enzyme site), gel electrophoresis, capillary array electrophoresis, MALDI-TOF MS, dynamic allele-specific hybridization, molecular beacons, restriction fragment length polymorphism, primer extension, temperature gradient gel electrophoresis, and the like.

In other embodiments, the cell viability after electroporation is at least 20, 25, 30, 35, 40, 45, 50, 55, 60, 65, 70, 75, 80, 85, or 95%. Cell viability can be measured by methods known in the art. For example, cells can be counted before and after electroporation by a cell counter apparatus. In other embodiments, apoptosis is measured. It is believed that introduction of large amounts of nucleic acids may induce apoptosis. It is contemplated that methods described herein will lead to less apoptosis than other methods in the art. In certain embodiments, the amount of cells exhibiting apoptosis after electroporation is less than 50, 45, 40, 35, 30, 25, 20, 15, 10, or 5%. Apoptosis refers to the specific process of programmed cell death and can be measured by methods known in the art. For example, apoptosis may be measured by Annexin V assays, activated caspase 3/7 detection assays, and Vybrant^{®} Apoptosis Assay (Life Technologies).

In further embodiments, the percentage of cells that express the nucleic acid encoding the marker is greater than about 10, 15, 20, 25, 30, 35, 40, 45, 50, 55, 60, 65, 70, 75, 80, 85, or 90%.

When a specific embodiment of the disclosure includes a range or specific value, as described herein, it is specifically contemplated that ranges and specific values (i.e. concentrations, lengths of nucleic acids, and percentages) may be excluded in embodiments of the invention. It is also contemplated that, when the disclosure includes a list of elements (e.g. cell types), embodiments of the invention may specifically exclude one or more elements in the list.

### VI. Examples

### Example 1 -

Cell culture: Cryopreserved PBMC were thawed and culture overnight in RPMI-1640+10% FBS+100u/ml rhIL-2+antibiotics. The attached cells in the tissue culture flask were removed. K562 were cultured in RPMI-1640+10% FBS+2mM L-glutamine+antibiotics. FibroblasrCells were in DMEM+10% FBS+antibiotics. Expanded T cells were activated by Dynalbeads Human T-Activator CD3/CD28 (Invitrogen, Carlsbad CA) following the protocol with the activation kit. Cells were transfected 3-6d post activation.

Electroporation: Cells were collected either directly for PBL, expanded T cells or K562, or with trypsinization for fibroblast. After washed with MXCT EP buffer, cells were mixed with mRNA (200ug/ml Cas9 and 100ug/ml gRNA, or 100ug/ml GFP) and/or single-stranded-DNA oligo (100ug/ml unless specified) and electroporated. Following 20min post EP incubation, cells were cultured for 2-5d before collecting cell pellet for gene modification asaay.

Genomic DNA extraction: Genomic DNA was extracted using Purelink genomic DNA Mini kit (Invitrogen, Carlsbad CA). The extracted genomic DNA was stored in -4C refrigerator before use.

Gene modification assay: Cel-1 assay was performed to assay the gene genomic DNA editing by using SURVEYOR Mutation detection kit (Trangenomic, Omaha NE). the protocol with the kit provided by the company was followed. The integration of HindIII recoganizing 6 nucleotides was assayed by HindIII digestion. The samples were analyzed with 10% TBE gel ((Invitrogen, Carlsbad CA).

CRISPR (Cas9 and gRNA): The whole kit for Cas9 and gRNA targeting to the specific 5' GGGGCCACTAGGGACAGGAT TGG 3' (SEQ ID NO:21) site on SSAV1 safe harbor site was purchased from Washington University in St. Luise Genome Engineering Center). The primers (F - 5' TTCGGGTCACCTCTCACTCC 3' (SEQ ID NO:22); R - 5' GGCTCCATCGTAAGCAAACC 3' (SEQ ID NO:23))for amplify genomic DNA segment containing the gRNA target site were included in the kit. The about 468bp amplicon was used for further Cel-1 assay and HindIII digest assay, the digestion of which will give two bands of about 170 and 298 bp each, if genomic DNA modification occurs.

CRISPR mRNA: the mRNA was made with mMESSAGE mMACHINE^{®} T7 Ultra Kit (Invitrogen, Carlsbad CA) from template plasmid DNA purchase from Washington University in St Luise.

Single-stranded Oligomer: the sequence of the oligos are as follows:

| Oligo size | Sequence | SEQ ID NO. |
|---|---|---|
| **100mer** | 5'TACTTTTATCTGTCCCCTCCACCCCACAGTGGGGCCA CTAGGGACAGAAGCTTGATTGGTGACAGAAAAGCCC CATCCTTAGGCCTCCTCCTTCCTAGTCTCC 3' | 24 |
| **70mer** | 5’CCTCCACCCCACAGTGGGGCCACTAGGGACAGAAG CTTGATTGGTGACAGAAAAGCCCCATCCTTAGGCC 3’ | 25 |
| **50mer** | 5'ACAGTGGGGCCACTAGGGACAGAAGCTTGATTGGT GACAGAAAAGCCCCA 3' | 26 |
| **26mer** | 5'CTAGGGACAG**AAGCTT**GATTGGTGAC 3' | 27 |

### Example 2

To validate that the methods are applicable to disease-associated genes, it was tested whether a restriction enzyme site could be integrated at the sickle-cell disease locus gene, *HBB.* K562 cells, a bone-marrow derived cell line from a patient with chronic myelogenous leukemia were cultured in RPMI-1640+10% FBS+2mM L-glutamine+antibiotics. Cells were then electroporated according to the method described in Example 1 with Cas9 plasmid for double strand DNA cut (Addgene plasmid #43945), a guide RNA plasmid targeting the Sickle cell disease (SCD) site (5' AGTCTGCCGTTACTGCCCTGTGG 3'(SEQ ID NO:28)), and the DNA donor sequence of single-stranded oligo for integration of Hind III restriction enzyme site (underlined):

(5'tgacacaactgtgttcactagcaacctcaaacagacaccatggtgcatctgactcctgAAGCTTggagaagtctgccgttact
gccctgtggggcaaggtgaacgtggatgaagttggtggtgaggccctgggcaggttggtatca 3'') (SEQ ID NO:29).

The gRNA template was made by PCR amplification with primers conjugated with T7 promoter. The primers were: Cel-1.F: 5- TTA ATACGACTCACTATAGGAGTCTGCCGTTACTGCCCTG -3 (SEQ ID NO:30) and Cel-1.R: 5- AAAAGCACCGACTCGGTGCC 3 (SEQ ID NO:31). Cas9 template was obtained by endonuclease-linearization (Xhol 1) of the Cas9 plasmid. mRNA was made by mMESSAGE mMACHINE T7 ULTRA Kit (Ambion). After electroporation of the cells, genomic DNA extractions and tests were performed as described in Example 1.

The integration specificity was first tested. As expected, an Oligo with gRNA Targeting AAVS1 Site was shown to integrate into the targeted AAVS1 site but not in the SCD locus (FIG. 14). It was next tested whether site-specific integration at the SCD locus was achieved. As shown in FIG. 15A-B, transfection with a guide RNA direct to the AAVS1 site and SCD locus resulted in successful genomic modification at these sites. FIG. 15B exemplifies that genomic DNA can be site-specifically modified at disease-associated loci.

### Example 3

The methods described herein may be used to correct the disease-causing mutation(s) in patient hematopoietic stem cells (HSC) to cure genetic diseases. This example describes methods to correct the mutation in Chronic Granulomatous Disease (CGD) for curing this disease. This disease will not only demonstrate the proof-of-concept of the gene therapy methods described herein, but also advance therapeutic approaches for this disease, which, thus far, is still an unmet challenge. Since the genetic mutations in CGD are well known, the techniques of in vitro functional assays of the cells with the disease are matured, the animal model of CGD is established, and low percentage of correction can lead to significant clinical efficacy. Non-viral approaches will be used to deliver messenger RNA (mRNA) encoding CRISPR (Cas9 and gRNA pair) and DNA oligomer targeting the most prevalent mutation ('hotspot') in the gp91phox gene located on Exon 7 at position 676 to convert the point mutation from 'T' (that results in disease phenotype) to 'C' (that is prevalent in normal cells). Delivery of the CRISPR will be facilitated by use of MaxCyte's cGMP and regulatory compliant close system flow electroporation platform.

If mutation in CGD as a model disease can be shown to be corrected in a clinical-relevant efficacy, this proof-of-concept of the approach described herein would in effect validate the technology platform as potentially curative therapy for expansion of the same approach to cure multiple other diseases where known mutations are associated with disease. Furthermore, since MaxCyte Flow Transfection System is a GMP-compliant, FDA-Master-File supported large volume transfection technology platform, and has been validated by current clinical trial and commercialization, success of this proposal study can be easily translated into clinical study and commercialization not only to CGD, but also to many other genetic diseases.

**HSC is the best choice for curing CGD and will be used in this proposal.** Gene therapy of correcting mutated gene in autologous HSC has the best potential to cure the genetic diseases. For CGD, HSC has been found clinically to be the right candidate for fighting this disease. So far, gene therapy for CGD using autologous HSC transduced by viral vector encoding for the disease gene/cDNA driven by a constitutive promoter have been tested clinically. This approach has demonstrated the feasibility of gene integration and expression from randomly integrated sites within the genome that has resulted in significant clinic benefit to patients even with <1-5% of cells expressing the corrected gene. However, a main concern regarding the conventional gene therapy is the risks of insertional mutagenesis due to the inability to control the location of gene integration into the genome, and the constitutive expression of cDNA in stem cells which will result in the cDNA expression to all sub lineages, even to those cells that may not express the gene when in normal health situation.

Non-viral approach has its advantage. However, the high cytotoxicity and low transfection efficiency of DNA plasmid transfection in most hematopoietic cells by non-viral transfection method hindered the non-viral transfection method from being used for HSC transfection. Through more than a decade of study, it was found that the high cytotoxicity and low transfection efficiency using electroporation for transfection are due to the DNA-uptake mediated apoptosis/pyroptosis, not the electroporation-mediated cell killing. To apply the non-viral transfection method for gene therapy, one has to find ways to efficiently transfect the transgene and improve the cell survival.

The mRNA transfection has been found to be an efficient way to express transgene with low cytotoxicity. The transient expression feature of mRNA transfection is advantageous for many applications, such as the forced expression of nucleases of CRISPR, TALEN or ZFN. The efficient specific gene editing in genomic DNA through electroporation of nuclease in mRNA formulation revitalizes the further application of this approach. A few successful approval of IND filing using nuclease electroporation in mRNA formulation are the good examples. For these current applications of mRNA transfection for clinical trials, DNA materials are intentionally avoided to lower cytotoxicity. Therefore the application is cleverly designed in the application area of gene knock out through gene indel. Current non-viral transfection is still not able to be applied in gene or nucleotide addition into genomic DNA.

The current finding described herein shows that, even though plasmid DNA uptake mediates high cytotoxicity, transfection of single stranded DNA oligomer does not induce cytotoxicity. This finding allows the non-viral approach to be used for gene correction of mono- or a few nucleotide mutations as an alternative way instead of constituitive expression of cDNA in gene therapy, which will significantly address the concern of mutagenesis and the non-wanted expression in certain sub-lineage worried in the current gene therapy approach. Since most genetic diseases involve mono- or a few nucleotide mutation, this gene correction approach may be very important and practical for fighting genetic diseases. Switching to nuclease transfection (CRSPR in this proposal as an example) in mRNA formulation and DNA single stranded oligomer as donor DNA for gene correction in CGD HSC offers minimal risk and high promising outcome not only for CGD, but also for gene therapy of other genetic disease.

### Research Design.

Genetic disease is an unmet challenge to our society. Most genetic diseases, such as CGD or sickle cell disease, have been treated with the only ability of controlling the symptom, such as using antibacterial/antifungal prophylaxis, IFN-r for CGD and blood transfusion for sickle cell disease. The lack of effective method of curing the diseases, patients with CGD unfortunately develop serious and even fatal recurrent infections, although the significant advancement made in antibiotic/antifugal therapy for CGD patients. Stem cell transplantation can cure the disease, but it requires the strictly matched donor, whom is difficult to find, limits the applicability.

Gene therapy using mutation-corrected autologous HSC ex vivo has been demonstrated the efficacy on benefiting patients, raising the hope to cure the genetic disease. So far, this approach, using virus as the gene delivery method, mostly used the whole cDNA encoding the mutated subunit with a promoter to constituitively express the therapeutic protein for the clinical trial. The safety of such approach with randomized integration in the whole genome and the constant expression for all the sub-lineages, some of which may not express the protein naturally, are the big concerns, leading to possible insertional mutagenesis and may be some unknown subsequence.

Non-viral approach for correcting the mutated nucleotides at the specific mutated site of genome of the autologous HSC will have less concern in insertional mutagenesis, gene expression silence, depletion of virus-infected cells, and the problem in gene expression regulation. However, the non-viral approach in gene therapy is unpopular so far, because the efficiency of non-viral approach was too low in both viability and transfection efficiency. However, by using nuclease (TALEN or CRISPR), recently Applicants found that the electroporation can mediated efficient nucleotide integration into targeted genome site, efficient correction of the specific mutated nucleotide, and efficient phenotype reverse from mutated cells to functional protein expressed cells in many different cell types, including HSC, without significant cytotoxicity, which is much higher in the efficiency than currently reported and therefore rekindles the hope of using non-viral approach for gene therapy. Additionally, the developed electroporation-based cGMP-compliant scalable gene delivery technology by MaxCyte can readily translate this finding into clinical trial and potentially commercialization.

Chronic Granulomatous Disease (CGD) is a group of hereditary diseases in which phagocytes do not produce reactive oxygen compounds (most importantly, the superoxide radical) used to kill certain pathogens. CGD affects about 1 in 200,000 people in the United States, with about 20 new cases diagnosed each year. Management of CGD involves early diagnosis, patient education and antibiotics for prophylaxis and treatment of infections. The morbidity of recurrent infections and inflammation is a major issue, with rates of infection around 0.3 per year. Hematopoietic stem cell (HSC) transplantation from a matched donor is curative but has significant associated risks (graft rejection, graft-versus-host disease, chemotherapy-related toxicities) and availability of matched donors. Gene therapy using autologous stem cells transduced by viral vector encoding for the disease gene/cDNA driven by a constitutive promoter have been tested clinically. This approach has demonstrated the feasibility of gene integration and expression from randomly integrated sites within the genome that has resulted in significant benefit to patients. However, a main concern regarding the conventional gene therapy is the risks of insertional mutagenesis due to the inability to control the location of gene integration into the genome.

The compositions and methods described herein may be used for targeted corrections of mutations in CGD patients. Messenger RNA based non-viral site-specific gene editing tools (CRISPR/Cas9 enzyme) with a correction sequence to specifically target the respective mutations in CGD patient HSCs may be used. Using the methods described herein, a non-viral, site-specific, ex vivo gene-modified cell therapy may be developed as a treatment for CGD.

**Develop protocols for site-specific correction of CGD mutations:** The first target correction will be the most prevalent mutation (a 'hotspot') in gp91phox in Exon 7 at position 676C to T by first using EBV-transformed B cells derived from patients. In this special CGD, correcting nucleotide "T" at amino acid site 226 to be "C" restores the site to be the right Arg from the stop codon, and expression from corrected cells can be quantified by the gp91 expression and confirmed by sequencing.

**Confirm functional gene correction in CGD patient HSC:** Autologous HSC from a CGD patient with the specific C676T mutation can be obtained. The transfection procedure can be optimized, and the mutated gene can be corrected using the methods described herein. The correction efficiency may be determined by the detection of gp91 expression and function restoration of superoxide production *in vitro,* followed by mice transplant studies in xenotransplant models to evaluate engraftment of such corrected patient HSCs and the restoration of function in human cells retrieved from the mice.

**Scale-up manufacturing process for clinical translation:** Autologous HSC from suitable CGD patients can be obtained. The mutated gene can then be corrected in scale-up cGMP-compliant manufacturing process. The correction efficiency and function restoration in vitro can then be checked.

With decades of study, Applicants and others found that DNA transfection is cytotoxic to most hematopoietic cells, which has been the most critic obstacle for preventing non-viral method to be effectively used in gene therapy. Applicants identified that DNA is the source for cytotoxicity, not the electroporation, as commonly intuitively believed. Finding the appropriate alternative for efficient transfection but with low cytotoxicity and high transfection efficiency has been our goal for a long time. Applicants are one of the first groups that pioneered the mRNA transfection and found that mRNA transfection meets the requirement. As shown in FIG. 17, morphologically, the flow electroporation transfection of HSC can mediate high transfection efficiency and low cytotoxicity by mRNA.

As shown in FIG. 10A-D, different mRNA concentrations, leading to much higher transfection efficiency than that by plasmid DNA, all result in higher cell viability than those with DNA plasmid trasnfection. GFP-mRNA transfection gave high viability (FIG. 10A), high transfection efficiency (FIG. 10C-D), the same cell proliferation rate relative to control cells (FIG. 10B), but DNA plasmid caused high cytotoxicity (FIG. 10A), retarded cell proliferation (FIG. 10B), and lower transfection efficiency (FIG. 10C-D).

Applicants further validated that not only mRNA is good for transfection, single-stranded DNA oligomer does not cause cytotoxicity either, which opens the possibility for gene correction by non-viral approach. As shown in FIG. 11, flow electroporation transfection of HSC with high transfection efficiency and low cytotoxicity by mRNA and single-stranded oligomer. HSC were transfected with MAXCYT flow electroporation technology. Control and GFP-mRNA transfection resulted in the similar viability (FIG. 11B), proliferation (FIG. 11C). The transfection of CRISPR (cas 9 (c) and gRNA (g)) and single-stranded oligomer (25mer, 50mer, 70mer and 100mer) all gave similar viability and proliferation, but a little lower than control cells. However, cells maintained high viability and proliferation (≥ 80% relative to control cells), demonstrating the high viability of c+g+olig transfection in HSC.

Not only mRNA transfection is low for cytotoxicity, it also mediates efficient function after transfection. As shown in FIG. 18, flow electroporation mediated efficient gene editing at AAVS1 site in HSC. Around 50% gene editing was achieved. Furthermore, the combination use of CRISPR and single-stranded DNA oligo in transfection can also mediate efficient nucleotide integration, a process of homologous recombination required for gene correction. As shown in FIG. 13A-B, flow electroporation mediated efficient nucleotide integration into AAVS1 site of HSC. The efficient nucleotide integration at specific genomic site of HSC is achieved. The integration of a 6-nucleotide Hind III recognizing sequence is oligomer size and concentration dependent. It can reach as high as around 40% integration in HSC.

Nucleotide integration by transfection of CRISPR and single-stranded DNA oligomer may be intuitively believed to be different from the nucleotide correction, which does not increase nucleotide length. The gene correction will further be different from the correction of the gene expression of the right functional protein. To further show by proof-of-concept, Applicants demonstrated that mRNA transfection of CRISPR and single-stranded oligomer not only can mediate gene integration, gene correction, but also can mediate phenotype reverse to have functional gene expression. As shown in FIG. 19, Flow electroporation mediated efficient restoration of gp91 expression in CGD patient EBV-transformed B cells. Actual rate of the HindIII-recognizing 6-neucleotide integration in the same EBV-transformed B cells was much higher than the rate of gp91 expressing cells (data not shown). This was also successfully performed in patient HSCs with high efficiency (>5%; data not shown).

The following methods may be used to correct CGD in patients: The most prevalent mutation (a 'hotspot') in gp91phox in Exon 7 at position 676C to T can first be targeted by first using EBV-transformed B cells derived from the patients. In this special CGD, correcting nucleotide "T" at amino acid site 226 to be "C" restores the site to be the right Arg from the stop codon, and expression from corrected cells can be quantified by the gp91 expression and confirmed by sequencing.

Four gRNA (g1, g2, g3, and g4) can be first tested and verified for efficacy. These gRNA are listed in the table below:

| | gRNA targeting to gp91 | SEQ ID NO: |
|---|---|---|
| g1 | TTTCCTATTACTAAATGATCNGG | 32 |
| g2 | CACCCAGATGAATTGTACGTNGG | 33 |
| g3 | TGCCCACGTACAATTCATCTNGG | 34 |
| g4 | AGTCCAGATCATTTAGTAATNGG | 35 |

The effect of oligomer size from 50mer to 200mer (As shown in the table below, 01-04) on the gene correction efficiency can be tested. It is expected that longer oligomer size may result in a better correction outcome, if the size still does not invoke the DNA sensor inside cells to initiate apoptosis or pyroptosis.

| | Single-Stranded Oligo for Gene Correction | SEQ ID NO: |
|---|---|---|
| 01 | 5'ACATTTTTCACCCAGATGAATTGTACGTGGGCAGACCGCAGAGAGTTTGGC-3' | 36 |
| 02 | 5'CTATTACTAAATGATCTGGACTTACATTTTTCACCCAGACGAATTGTACGTGG GCAGACCGCAGAGAGTTTGGCTGTGCATAATATAACAGTTTGTGAA-3' | 37 |
| 03 | 5'TCTTTTAATAAAACAATTTAATTTCCTATTACTAAATGATCTGGACTTACATTT TTCACCCAGATGAATTGTACGTGGGCAGACCGCAGAGAGTTTGGCTGTGCATAA TATAACAGTTTGTGAACAAAAAATCTCAGAATGGGGAA-3 | 38 |
| 04 | 5'CAGAGCACTTAAAATATATGCAGAATCTTTTAATAAAACAATTTAATTTCCTA TTACTAAATGATCTGGACTTACATTTTTCACCCAGATGAATTGTACGTGGGCAG ACCGCAGAGAGTTTGGCTGTGCATAATATAACAGTTTGTGAACAAAAAATCTCA GAATGGGGAAAAATAAAGGAATGCCCAATCCCTCA-3 | 39 |

For the first phase of study, oligomer can be used to integrate a HindIII-recognizing site (AAGCTT), and the efficiency of targeting of the four gRNA can be tested using an Indel efficiency by Cel-1 assay and integration efficiency of HindIII-recognizing site by HindIII digesting assay of the PCR amplified amplicon. Oligomer with the HindIII-recognizing sequence removed can then be used, and with T to C change at the mutation site to test the restoration of the gp91 expression for extended long time to understand the persistency of gene correction.

To confirm the true correction, the three assays listed below may be used: 1) Use antibody against gp91 to assay to check the restoration of gp91 expression; 2) Sorting gp91 positive cells, and sequencing the PCR amplicon to verify the correction; and 3) *In vitro* functional study of O2⁻ production by measure the enhanced chemiluminescence with the stimulation of phorbol myristate acetate (PMA).

Since Applicants already have Cas9 produced and tested, Applicants do not expect any problems relating to obtaining efficient gene editing at the site close to the mutation site. A cel-1 assay can be used to check the efficiency of gene editing. A Hind III recognizing site may also be incorporated into the mutation site to check oligomer integration. With the methods and data described previously, very promising results were found with the two tested gRNA-2 with 100mer sized oligomer. The gp91 gene expression was restored at a level of more than 3%. Different structured oligomers may be designed, if necessary. These include oligomers which have bond modification for oligomer stability inside cells, or even double stranded oligomer. With the further optimization, it is believed that 5-10% of correction efficiency may be achived, the level of which may be high enough to see significant clinic benefit for CGD patients.

**Confirm functional gene correction in CGD patient HSC:** Autologous HSC from a CGD patient with the specific C676T mutation can be obtained. The transfection can be optimized, and the mutated gene can be corrected using the four gRNA and the oligomer described above. The correction efficiency by detection of gp91 expression and function restoration of superoxide production in vitro can then be tested.

Once one has achieved the restoration of gp91 expression at about 5-10%, SCID mice engraftment studies can be done in xenotransplant models to evaluate engraftment of such corrected patient HSCs for about 1-4 month engraftment duration. Furthermore, the restoration of function in human cells retrieved from the mice can also be tested. The efficiency of the engraftment of the corrected HSC with i.v. tail vein injection of 1e6-5e6/mice with duration of 1-3 months can also be tested.

To confirm gene correction, the following studies can be used: 1) Use antibody against gp91 to assay the restoration of gp91 expression; 2) Sorting gp91 positive cells, and sequencing the PCR amplicon to verify the correction; 3) *In vitro* functional study of O2⁻ production by measure the enhanced chemiluminescence with the stimulation of phorbol myristate acetate (PMA) with the 14-17d differentiated myloid cells; 4) *In vitro* FACS analysis of the functional study of O2⁻ production using dihydrohodamine 123 (DHR) fluorescence probe after PMA stimulation with 14-17d differentiated myeloid cells; 5) *In vitro* functional study of superoxide O2⁻ production in forming the reduced formazan from nitroblue tetrazolium (NBT) after PMA stimulation of the differentiated myeloid colony in semisolid agarose; and 6) *In vitro* functional FACS study of superoxide O2⁻ production of the differentiated myeloid retrieved from the engrafted HSC in SCID mice, using (DHR) fluorescence probe after PMA stimulation.

Applicants are very experienced in CD34 HSC in HSC culture, transfection and gene editing. Transfection conditions and transfection time point after thawing and culturing with cytokines may further be optimized. The gene editing can be checked, and the oligomer may be modified to incorporate Hind III recognizing nucleotides to check the efficiency of nucleotide integration at the mutation site. Oligomer size and concentration and CRISPR ratio and concentration may be optimized to obtain efficient gene editing and Hind III integration. The Hind III-recognizing site integration can be correlated with the mutated gene correction. It may be necessary or advantageous to culture the HSC for 1 to 5d after thawing to allow HSC to be into full proliferate phase in order to have efficient correction efficiency. Since Applicants already see > 6% restoration of gp91 expression in differentiated neutrophils from corrected CGD-patient HSC, no problems are expected.

Scale-up manufacturing process for clinical translation: Autologous HSC from suitable CGD patients can be obtained. It is expected that 4e8-4e9 HSC will be needed for clinical trial for each individual. The scale up procedures and cell handling for the preparation of the future clinical trial can then be studied. The scale up may start from 2e6 HSC to 1e8 HSC, then from 1e8 to 1e9 or 4e9. The transfected cells will be assayed *in vitro.* The mutated gene may then be corrected in scale-up cGMP-compliant manufacturing process. The correction efficiency and function restoration in vitro can then be evaluated. SCID mice engraftment study in xenotransplant models to evaluate engraftment of such corrected patient HSCs may also be performed.

The following experiments may be used to confirm gene correction: 1) Use antibody against gp91 to assay the restoration of gp91 expression; 2) Sorting gp91 positive cells, and sequencing the PCR amplicom to verify the correction; 3) *In vitro* functional study of O2⁻ production by measure the enhanced chemiluminescence with the stimulation of phorbol myristate acetate (PMA) with the 14-17d differentiated myloid cells; 4) *In vitro* FACS analysis of the functional study of O2⁻ production using dihydrohodamine 123 (DHR) fluorescence probe after PMA stimulation with 14-17d differentiated myloid cells; 5) *In vitro* functional study of superoxide O2⁻ production in forming the reduced formazan from nitroblue tetrazolium (NBT) after PMA stimulation of the differentiated myeloid colony in semisolid agarose; and 6) *In vitro* functional FACS study of superoxide O2⁻ production of the differentiated myeloid retrieved from the engrafted HSC in SCID mice, using (DHR) fluorescence probe after PMA stimulation.

The methods described herein can be used to develop a translational platform technology for manufacturing a large number of autologous HSC with efficient mutation correction for treating CGD, as an example platform for genetic diseases. The results from the proposed studies will be used to develop technology transfer package, including development of manufacturing processes, analytical methodologies and product characterization and release testing requirements for translation into IND-enabling studies at the NIH cGMP facility to support filing of IND application for conduct of human clinical trials. The methods described in this example can also be used to develop a scalable process for manufacture of clinically relevant numbers of gene-corrected autologous HSC for treating CGD. This project will produce mutation-corrected autologous HSC with high viability, low toxicity and clinically relevant levels of gene-correction. Furthermore, these results may further validate application of the developed approach for treatment other genetic diseases and warrant separate further investigations.

## Claims

1. A method for site-specific sequence modification of a target genomic DNA region in mammalian hematopoietic stem cells (HSCs) isolatable from the body of a subject comprising:
transfecting the hematopoietic stem cells (HSCs) by flow electroporation using a flow electroporation device with a non-viral composition comprising (a) a single-stranded DNA oligo, wherein the DNA oligo is from about 40 to about 100 nucleic acids and the concentration of the DNA oligo in the composition is at least 100 µg/ml; (b) an RNA encoding the site-specific nuclease Cas9; and (c) a guide RNA
wherein the DNA oligo comprises:
(i) a homologous region comprising DNA sequence homologous to the target genomic DNA region; and
(ii) a sequence modification region; and wherein the genomic DNA sequence is modified specifically at the target genomic DNA region;
and wherein said stem cells are no human embryonic stem cells.

2. The method of claim 1wherein the oligo comprises at least 10 nucleic acids of homologous sequence, at least 20 nucleic acids of homologous sequence, or at least 30 nucleic acids of homologous sequence.

3. The method of any one of claims 1-2, wherein the efficiency of the sequence modification is greater than 3%, greater than 5%, or greater than 10%.

4. The method of any one of claims 1-3, wherein the cell viability after electroporation is at least 30%, at least 40%, or at least 50%.

5. The method of any one of claims 1-4, wherein the composition comprises two or more DNA oligos with different homologous sequences, or wherein the composition comprises two or more DNA oligos with different homologous sequences and two or more DNA digesting agents, in particular wherein the DNA digesting agents are targeted to different genomic sites.

6. The method of any one of claims 1-5 wherein the cells are cells isolated from a patient, were isolated from the patient at a time period of less than one week prior to transfection of the cells, or were isolated from the patient at a time period of less than one day prior to transfection of the cells, in particular wherein the isolated cells have not been frozen, and/or wherein the isolated cells comprise two or more different cell types, and/or wherein the two or more different cell types comprise two or more cell types at different stages of pluripotency.

7. The method of any one of claims 1-6, wherein the efficiency of the sequence modification is greater than 3%, greater than 5%, greater than 10%, and/or wherein the cell viability after electroporation is at least 30%, at least 40%, or at least 50%.

8. The method of any one of claims 1-7, wherein the cells are isolated from the bone marrow of the subject, in particular wherein the stem cells comprise hematopoietic stem cells, in particular wherein the hematopoietic stem cells comprise the cell surface marker CD34+.

9. The method of any of claims 1-8, further comprising expanding a clonal isolated and selected cell to produce clonal cells having the DNA sequence modification, in particular wherein the clonal cells are expanded for large scale manufacturing, more in particular wherein the clonal cells are expanded in a volume greater than 1 L, or in a volume of 3 L or more.

10. The method of any of claims 1-9, wherein the cells are cultured in serum-free media.

11. The method of any of claims 1-10, wherein
i) the DNA sequence modification is one or more stop codons, changes one or more base pairs of the genomic sequence, adds one or more base pairs of the genomic sequence, or deletes one or more base pairs of the genomic sequence; and/or
ii) the method further comprises screening the cells for the sequence modification, and/or further comprises freezing transfected cells, and/or further comprises expanding transfected cells that were previously frozen.

12. A method for producing a stable cell line comprising a genomic DNA sequence modification of a target genomic DNA sequence, the method comprising: transfecting mammalian hematopoietic stem cells (HSCs) isolatable from the body of a subject by flow electroporation with a non- viral composition comprising (a) a single-stranded DNA oligo, wherein the DNA oligo is from about 40 to about 100 nucleic acids and the concentration of the DNA oligo in the composition is at least 100 µg/ml; (b) an RNA encoding the site specific nuclease Cas9 ; and (c) a guide RNA; wherein the DNA oligo comprises:
i) a homologous region comprising nucleic acid sequence homologous to the target genomic DNA region; and
ii) a sequence modification region, and wherein said stem cells are no human embryonic stem cells; and screening transfected cells for the genomic DNA sequence modification at the target genomic DNA region; isolating screened transfected cells by limiting dilution to obtain clonal cells; expanding isolated transfected cells to produce a stable cell line comprising the genomic DNA sequence modification.

## Patentansprüche

1. Verfahren zur ortsspezifischen Sequenzmodifikation einer genomischen Ziel-DNA-Region in hämatopoetischen Stammzellen (HSCs) von Säugetieren, die aus dem Körper eines Subjekts isolierbar sind, umfassend:
Transfektion der hämatopoetischen Stammzellen (HSCs) durch Fließelektroporation unter Verwendung einer Fließelektroporationsvorrichtung mit einer nicht-viralen Zusammensetzung, umfassend (a) ein einzelsträngiges DNA-Oligo, wobei das DNA-Oligo aus etwa 40 bis etwa 100 Nukleinsäuren besteht und die Konzentration des DNA-Oligos in der Zusammensetzung mindestens 100 µg/ml beträgt; (b) eine RNA, die für die ortsspezifische Nuklease Cas9 kodiert; und (c) eine Leit-RNA
wobei das DNA-Oligo umfasst:
(i) eine homologe Region, die eine zur genomischen Ziel-DNA-Region homologe DNA-Sequenz umfasst; und
(ii) eine Sequenzmodifikationsregion; und wobei die genomische DNA-Sequenz spezifisch in der genomischen Ziel-DNA-Region modifiziert wird;
und wobei diese Stammzellen keine menschlichen embryonalen Stammzellen sind.

2. Verfahren nach Anspruch 1, wobei das Oligo mindestens 10 Nukleinsäuren mit homologer Sequenz, mindestens 20 Nukleinsäuren mit homologer Sequenz oder mindestens 30 Nukleinsäuren mit homologer Sequenz umfasst.

3. Verfahren nach einem der Ansprüche 1-2, wobei die Effizienz der Sequenzmodifikation größer als 3 %, größer als 5 % oder größer als 10 % ist.

4. Verfahren nach einem der Ansprüche 1-3, wobei die Lebensfähigkeit der Zellen nach der Elektroporation mindestens 30%, mindestens 40% oder mindestens 50% beträgt.

5. Verfahren nach einem der Ansprüche 1-4, wobei die Zusammensetzung zwei oder mehr DNA-Oligos mit unterschiedlichen homologen Sequenzen umfasst, oder wobei die Zusammensetzung zwei oder mehr DNA-Oligos mit unterschiedlichen homologen Sequenzen und zwei oder mehr DNA-Verdauungsmittel umfasst, insbesondere wobei die DNA-Verdauungsmittel auf unterschiedliche genomische Stellen ausgerichtet sind.

6. Verfahren nach einem der Ansprüche 1-5, wobei es sich bei den Zellen um Zellen handelt, die aus einem Patienten isoliert wurden, die in einem Zeitraum von weniger als einer Woche vor der Transfektion der Zellen aus dem Patienten isoliert wurden, oder die in einem Zeitraum von weniger als einem Tag vor der Transfektion der Zellen aus dem Patienten isoliert wurden, insbesondere wobei die isolierten Zellen nicht eingefroren wurden, und/oder wobei die isolierten Zellen zwei oder mehr verschiedene Zelltypen umfassen, und/oder wobei die zwei oder mehr verschiedenen Zelltypen zwei oder mehr Zelltypen in verschiedenen Pluripotenzstadien umfassen.

7. Verfahren nach einem der Ansprüche 1-6, wobei die Effizienz der Sequenzmodifikation größer als 3 %, größer als 5 %, größer als 10 % ist und/oder wobei die Lebensfähigkeit der Zellen nach der Elektroporation mindestens 30 %, mindestens 40 % oder mindestens 50 % beträgt.

8. Verfahren nach einem der Ansprüche 1-7, wobei die Zellen aus dem Knochenmark des Subjekts isoliert werden, insbesondere wobei die Stammzellen hämatopoetische Stammzellen umfassen, insbesondere wobei die hämatopoetischen Stammzellen den Zelloberflächenmarker CD34+ umfassen.

9. Verfahren nach einem der Ansprüche 1-8, ferner umfassend das Expandieren einer klonalen isolierten und ausgewählten Zelle, um klonale Zellen mit der DNA-Sequenzmodifikation zu erzeugen, wobei die klonalen Zellen insbesondere für die Herstellung im großen Maßstab expandiert werden, insbesondere wobei die klonalen Zellen in einem Volumen von mehr als 1 Liter oder in einem Volumen von 3 Liter oder mehr expandiert werden.

10. Verfahren nach einem der Ansprüche 1-9, wobei die Zellen in serumfreien Medien kultiviert werden.

11. Das Verfahren nach einem der Ansprüche 1-10, wobei
i) die DNA-Sequenzmodifikation ein oder mehrere Stoppcodons ist, ein oder mehrere Basenpaare der genomischen Sequenz verändert, ein oder mehrere Basenpaare der genomischen Sequenz hinzufügt oder ein oder mehrere Basenpaare der genomischen Sequenz entfernt; und/oder
ii) das Verfahren ferner das Screening der Zellen auf die Sequenzmodifikation umfasst, und/oder ferner das Einfrieren transfizierter Zellen umfasst, und/oder ferner das Expandieren transfizierter Zellen, die zuvor eingefroren wurden, umfasst.

12. Verfahren zur Herstellung einer stabilen Zelllinie, die eine genomische DNA-Sequenzmodifikation einer genomischen Ziel-DNA-Sequenz umfasst, wobei das Verfahren umfasst: Transfektion von hämatopoetischen Stammzellen (HSCs) von Säugetieren, die aus dem Körper eines Subjekts isolierbar sind, durch Fließelektroporation mit einer nicht-viralen Zusammensetzung, umfassend (a) ein einzelsträngiges DNA-Oligo, wobei das DNA-Oligo aus etwa 40 bis etwa 100 Nukleinsäuren besteht und die Konzentration des DNA-Oligos in der Zusammensetzung mindestens 100 µg/ml beträgt; (b) eine RNA, die für die ortsspezifische Nuklease Cas9 kodiert; und (c) eine Leit-RNA; wobei das DNA-Oligo umfasst:
i) eine homologe Region, die eine Nukleinsäuresequenz umfasst, die zu der genomischen Ziel-DNA-Region homolog ist; und
ii) eine Sequenzmodifikationsregion, und wobei die Stammzellen keine humanen embryonalen Stammzellen sind; und Screenen der transfizierten Zellen auf die genomische DNA-Sequenzmodifikation an der genomischen DNA-Zielregion; Isolieren der gescreenten transfizierten Zellen durch begrenzende Verdünnung, um klonale Zellen zu erhalten; Expandieren der isolierten transfizierten Zellen, um eine stabile Zelllinie zu erzeugen, die die genomische DNA-Sequenzmodifikation umfasst.

## Revendications

1. Procédé de modification de séquence site-spécifique d'une région d'ADN génomique cible dans des cellules souches hématopoïétiques (CSH) de mammifère pouvant être isolées du corps d'un sujet comprenant :
la transfection des cellules souches hématopoïétiques (CSH) par électroporation en flux en utilisant un dispositif d'électroporation en flux avec une composition non virale comprenant (a) un oligo d'ADN simple brin, dans lequel l'oligo d'ADN provient d'environ 40 à environ 100 acides nucléiques et la concentration de l'oligo d'ADN dans la composition est d'au moins 100 µg/ml ; (b) un ARN codant pour la nucléase site-spécifique Cas9 ; et (c) un ARN guide dans lequel l'oligo d'ADN comprend :
(i) une région homologue comprenant une séquence d'ADN homologue à la région d'ADN génomique cible ; et
(ii) une région de modification de séquence ; et dans lequel la séquence d'ADN génomique est modifiée spécifiquement au niveau de la région d'ADN génomique cible ;
et dans lequel lesdites cellules souches sont des cellules souches embryonnaires non humaines.

2. Procédé selon la revendication 1, dans lequel l'oligo comprend au moins 10 acides nucléiques de séquence homologue, au moins 20 acides nucléiques de séquence homologue, ou au moins 30 acides nucléiques de séquence homologue.

3. Procédé selon l'une quelconque des revendications 1 à 2, dans lequel l'efficacité de la modification de séquence est supérieure à 3 %, supérieure à 5 %, ou supérieure à 10 %.

4. Procédé selon l'une quelconque des revendications 1 à 3, dans lequel la viabilité cellulaire après l'électroporation est d'au moins 30 %, d'au moins 40 %, ou d'au moins 50 %.

5. Procédé selon l'une quelconque des revendications 1 à 4, dans lequel la composition comprend deux oligos d'ADN ou plus avec différentes séquences homologues, ou dans lequel la composition comprend deux oligos d'ADN ou plus avec différentes séquences homologues et deux agents de digestion de l'ADN ou plus, en particulier dans lequel les agents de digestion de l'ADN sont ciblés vers des sites génomiques différents.

6. Procédé selon l'une quelconque des revendications 1 à 5, dans lequel les cellules sont des cellules isolées chez un patient, ont été isolées chez le patient à une période de moins d'une semaine avant la transfection des cellules, ou ont été isolées chez le patient à une période de moins d'un jour avant la transfection des cellules, en particulier dans lequel les cellules isolées n'ont pas été congelées, et/ou dans lequel les cellules isolées comprennent deux types cellulaires différents ou plus, et/ou dans lequel les deux types cellulaires différents ou plus comprennent deux types cellulaires ou plus à différents stades de pluripotence.

7. Procédé selon l'une quelconque des revendications 1 à 6, dans lequel l'efficacité de la modification de séquence est supérieure à 3 %, supérieure à 5 %, supérieure à 10 %, et/ou dans lequel la viabilité cellulaire après l'électroporation est d'au moins 30 %, d'au moins 40 %, ou d'au moins 50 %.

8. Procédé selon l'une quelconque des revendications 1 à 7, dans lequel les cellules sont isolées à partir de la moelle osseuse du sujet, en particulier dans lequel les cellules souches comprennent des cellules souches hématopoïétiques, en particulier dans lequel les cellules souches hématopoïétiques comprennent le marqueur de surface cellulaire CD34+.

9. Procédé selon l'une quelconque des revendications 1 à 8, comprenant en outre l'expansion d'une cellule clonale isolée et sélectionnée afin de produire des cellules clonales comportant la modification de séquence d'ADN, en particulier dans lequel les cellules clonales sont soumises à une expansion pour une fabrication à grande échelle, plus en particulier dans lequel les cellules clonales sont soumises à une expansion dans un volume supérieur à 1 I, ou dans un volume de 3 I ou plus.

10. Procédé selon l'une quelconque des revendications 1 à 9, dans lequel les cellules sont mises en culture dans un milieu exempt de sérum.

11. Procédé selon l'une quelconque des revendications 1 à 10, dans lequel
i) la modification de séquence d'ADN est un ou plusieurs codons d'arrêt, des modifications d'une seule ou de plusieurs paires de bases de la séquence génomique, l'addition d'une seule ou de plusieurs paires de bases de la séquence génomique, ou la délétion d'une seule ou de plusieurs paires de bases de la séquence génomique ; et/ou
ii) le procédé comprend en outre le criblage des cellules pour la modification de séquence, et/ou comprend en outre la congélation des cellules transfectées, et/ou comprend en outre l'expansion des cellules transfectées qui ont été précédemment congelées.

12. Procédé de production d'une lignée cellulaire stable comprenant une modification de séquence d'ADN génomique d'une séquence d'ADN génomique cible, le procédé comprenant : la transfection de cellules souches hématopoïétiques (CSH) de mammifère pouvant être isolées du corps d'un sujet par électroporation en flux avec une composition non virale comprenant (a) un oligo d'ADN simple brin, dans lequel l'oligo d'ADN provient d'environ 40 à environ 100 acides nucléiques et la concentration de l'oligo d'ADN dans la composition est d'au moins 100 µg/ml ; (b) un ARN codant pour la nucléase site-spécifique Cas9 ; et (c) un ARN guide ; dans lequel l'oligo d'ADN comprend :
i) une région homologue comprenant une séquence d'acide nucléique homologue à la région d'ADN génomique cible ;
ii) une région de modification de séquence, et dans lequel lesdites cellules souches sont des cellules souches embryonnaires non humaines ; et le criblage des cellules transfectées pour la modification de séquence d'ADN génomique au niveau de la région d'ADN génomique cible ; l'isolement des cellules transfectées criblées par dilution limite pour obtenir des cellules clonales ; l'expansion des cellules transfectées isolées afin de produire une lignée cellulaire stable comprenant la modification de séquence d'ADN génomique.
